# EUROPEAN PATENT SPECIFICATION

(11) **EP 2 681 311 B1**
(45) Date of publication and mention of the grant of the patent: **28.03.2018**
(21) Application number: 12703231.6
(22) Date of filing: 31.01.2012
(51) Int. Cl.: C12N 5/076, A61K 31/59, A61K 35/52

(54) **ACTIVE VITAMIN D OR ANALOGS THEREOF FOR USE IN IN VITRO FERTILIZATION**
AKTIVES VITAMIN D ODER ANALOGA DAVON ZUR VERWENDUNG BEI DER IN-VITRO-BEFRUCHTUNG
VITAMINE D ACTIVE OU SES ANALOGUES POUR L'UTILISATION DANS LA FÉCONDATION IN VITRO

(30) Priority: 28.02.2011 EP 11156230; 20.12.2011 EP 11194545
(43) Date of publication of application: 08.01.2014
(73) Proprietor: Rigshospitalet, Copenhagen University Hospital, 2100 Copenhagen Ø (DK)
(72) Inventor: JENSEN, Martin Blomberg, DK-2100 Copenhagen Ø (DK)
(74) Representative: Plougmann Vingtoft a/s
(86) International application number: PCT/DK2012/050040
(87) International publication number: WO 2012/116699

(56) References cited:
- WO-A1-2007/020042
- A Tiwari: "Elocalcitol, a vitamin D3 analog for the potential treatment of benign prostatic hyperplasia, overactive bladder and male infertility", idrugS THE INVESTIGATIONAL DRUG S JOURNAL, 1 July 2009 (2009-07-01), XP055008575, Retrieved from the Internet: URL:http://thomsonreuters.com/content/scie nce/pdf/idrugs-contents.pdf [retrieved on 2011-09-30]
- AQUILA SAVERIA ET AL: "Human sperm anatomy: ultrastructural localization of 1alpha,25-dihydroxyvitamin D receptor and its possible role in the human male gamete.", JOURNAL OF ANATOMY NOV 2008 LNKD- PUBMED:19014363, vol. 213, no. 5, November 2008 (2008-11), pages 555-564, XP002641183, ISSN: 1469-7580
- DATABASE WPI Week 200767 Thomson Scientific, London, GB; AN 2007-712457 XP002641184, -& JP 2007 215511 A (CHUGAI PHARM CO LTD) 30 August 2007 (2007-08-30)
- BLOMBERG JENSEN MARTIN ET AL: "Vitamin D receptor and vitamin D metabolizing enzymes are expressed in the human male reproductive tract.", HUMAN REPRODUCTION (OXFORD, ENGLAND) MAY 2010 LNKD- PUBMED:20172873, vol. 25, no. 5, May 2010 (2010-05), pages 1303-1311, XP002641182, ISSN: 1460-2350
- JENSEN M BLOMBERG ET AL: "Vitamin D and sperm function: CYP24A1 may serve as a novel marker of semen quality", INTERNATIONAL JOURNAL OF ANDROLOGY, vol. 33, no. Suppl. 1, October 2010 (2010-10), pages 47-48, XP008137806, & 6TH EUROPEAN CONGRESS OF ANDROLOGY; ATHENS, GREECE; SEPTEMBER 29 OCTOBER 01, 2010 ISSN: 0105-6263
- DONNELLY E T ET AL: "In vitro fertilization and pregnancy rates: the influence of sperm motility and morphology on IVF outcome.", FERTILITY AND STERILITY AUG 1998 LNKD- PUBMED:9696226, vol. 70, no. 2, August 1998 (1998-08), pages 305-314, XP002641185, ISSN: 0015-0282
- "European Association of Urology Guidelines on Male infertility: The 2012 update, Jungwirth A et al", 2012
- "Andrology: Male reproductive health and dysfunction, 2010, Nieschlag E", 2010
- LIU AND BAKER HUM REPROD 2000 15:702-8: HUM REPROD, 2000,
- LIU AND BAKER HUM REPROD 1999 DEFECTIVE SPERM-ZONA PELLUCIDA INTERACTION: HUM REPROD, 1999,
- BLOMBERG JENSEN M NATURE REVIEWS ENDOCRINOLOGY 2014: NATURE REVIEWS ENDOCRINOLOGY, 2014,

## Description

### Technical field of the invention

The present disclosure relates to the discovery of a positive role for active vitamin D (calcitriol) or active derivatives thereof in different in vivo and in vitro fertilization techniques (IVF), e.g. due to in improved sperm-egg binding. Thus, in here is disclosed media comprising active vitamin D and methods of use of such media.

### Background of the invention

The vitamin D receptor (VDR), and the vitamin D (VD) metabolizing enzymes are expressed in the human ejaculatory duct, germ cells and mature spermatozoa. The importance of vitamin D for male reproduction has been demonstrated in several animal studies. One of the VDR knock-out mice models is characterized by an infertile phenotype, and VD deficiency in rodents leads to reduced sperm counts, impaired sperm motility and lower fertility rates in females inseminated with semen from VD deficient males (Kwiecinski et al., 1989). In humans, VDR and all the VD metabolizing enzymes are co-expressed during the late stages of spermatogenesis and in the neck of mature spermatozoa. Optimal sperm function may thus depend on a direct effect of VD, however, it could also be influenced indirectly through calcium homeostasis since the impaired fertility in animal models was partly restored solely by normalization of serum calcium levels. VD has widespread biological functions, including an essential role for systemic calcium homeostasis and a role for calcium in the maturation of human spermatozoa is well documented and highlighted by the 2-3 fold higher calcium concentration in human epididymal and prostate fluid compared to serum. VD has been hypothesized to be important for the proposed trans-epithelial calcium transfer in the epididymis.

The effects of VD rely on activation of cholecalciferol (inactive vitamin D3), which normally starts in the skin, where UV-B irradiation converts 7-dehydrocholesterol to Vitamin D3. It is subsequently activated by the hepatic 25-hydroxylases (CYP2R1, CYP27A1) and the renal 1α-hydroxylase (CYP27B1), before the active 1,25(OH)2D3 (calcitriol) binds with high affinity to the VDR until inactivated by CYP24A1. 1,25(OH)2D3 exerts both genomic and non-genomic actions through binding to VDR (Norman, 1998), but the precise effects of Vitamin D in human spermatozoa remains to be shown. Vitamin D status in humans is generally monitored by measuring serum 25-hydroxyvitamin D levels, and there is a high frequency of VD insufficiency (< 50 nM) among otherwise healthy adults in the western world.

The spermatozoa of mammals, including humans, are in a protective, nonlabile state at ejaculation and are incapable of fertilization even if they are placed in direct contact with an oocyte. Consequently, they must undergo a subsequent period of final maturation during which they acquire the capacity to interact with the oocyte and achieve fertilization. This process is termed capacitation, and spermatozoa in the ejaculate are prevented from undergoing capacitation by one or more decapacitation factors that are present in the seminal plasma. Capacitation of spermatozoa is essential for fertilization not only in vivo but also in vitro, and underlies the manipulation of spermatozoa for clinical in vitro fertilization (IVF). High quality spermatozoa are thus separated from the seminal plasma and dead cells during sperm preparation for IVF by laboratory procedures such as percoll gradient centrifugation or swim up. The spermatozoa are hereafter resuspended in media comprising e.g. fructose or glucose for nutrition and calcium, bicarbonate and bovine serum albumin (BSA) which all promotes the capacitation process that allows fertilization of the oocyte. The oocyte and the spermatozoa are placed in another media containing various nutritional factors, salts and growth factors needed for the proliferation of the fertilized oocyte. US 2002/0028509 A1 discloses different media relating to IVF, and thus relates to the same technical field as the present invention. However, US 2002/0028509 A1 is silent about active vitamin D (calcitriol).

Aquila Saveria et al. (Human sperm anatomy: ultrastructural localization of 1 alpha,25-dihydroxyvitamin D receptor and its possible role in the human male gamete.", JOURNAL OF ANATOMY NOV 2008, vol. 213, no. 5, November 2008 (2008-11), pages 555-564), shows that 1,25(OH)2D3 increases tyrosine phosphorylation and cholesterol efflux, but states that the physiological significance and specific roles of 1,25(OH)2D3/VDR in the human male gamete require further investigation.

### Summary of the invention

VDR and the VD metabolizing enzymes are expressed in mature spermatozoa, and in vitro experiments revealed that active vitamin D (calcitriol) through non-genomic VDR activation increased transiently intracellular calcium concentration from an intracellular calcium storage in the neck of human spermatozoa, induced sperm motility, the acrosome reaction and promoted binding between the oocyte and the spermatozoa. Thus, the present inventors have discovered a direct effect of 1,25(OH)2D3 (calcitriol) on intracellular calcium level, motility and acrosome reaction in mature spermatozoa. Furthermore, the increase in calcium facilitates binding to the oocyte as shown by the improved egg-sperm binding after exposure to media containing activated vitamin D.

Hence, improved media for different uses in IVF would be advantageous, and in particular a more efficient and/or reliable method for improving fertilization of eggs in IVF would be advantageous.

Thus, one aspect of the invention relates to a method for improving the ability of a spermatozoa to fertilize an egg in vitro, said method comprising
- providing a medium comprising active vitamin D (calcitriol) or analogs thereof,
- providing a sample of spermatozoa, and
- incubating said sample of spermatozoa with said medium;
wherein said improved ability of a spermatozoa to fertilize an egg is at least partly due to an increased binding of spermatozoa to the egg;
wherein said analogs are agonists which can bind and release the non-genomic effect via VDR (Vitamin D receptor) in spermatozoa OR wherein said analogs are selected from the group consisting of the list of table 1 and 1,25(OH)₂ Lumisterol₃ (JN) and 25-OHD₃;
wherein said active vitamin D or analogs thereof is present at a concentration of 10⁻¹⁵ - 10⁻⁴ M.

Another aspect of the invention relates to the use of a composition comprising active vitamin D (calcitriol) or analogs thereof for, in vitro, improving the ability of a spermatozoa to bind to an egg;
wherein said analogs are agonists which can bind and release the non-genomic effect via VDR (Vitamin D receptor) in spermatozoa OR wherein said analogs are selected from the group consisting of the list of table 1 and 1,25(OH)₂ Lumisterol₃ (JN) and 25-OHD₃;
wherein said active vitamin D or analogs thereof is present at a concentration of 10⁻¹⁵ - 10⁻⁴ M.

Another disclosure of the present application relates to a method for improving in vitro fertilization of an egg, said method comprising
- providing a medium comprising active vitamin D (calcitriol) or analogs thereof,
- providing a sample of spermatozoa,
- providing a sample comprising one or more eggs, and
- incubating said sample of spermatozoa and said sample of one or more human eggs in said medium.

Yet a disclosure of the present application is the use of a medium comprising active vitamin D (calcitriol) or analogs thereof, for improving in vivo or in vitro fertilization of an egg.

Another disclosure of the present application relates to the use of a medium comprising active vitamin D (calcitriol) or analogs thereof, for improving the ability of a spermatozoa to fertilize an egg in vivo or in vitro.

A further disclosure of the present application relates to a medium comprising active vitamin D (calcitriol) or analogs thereof for use in the treatment of infertility in a male or female subject.

Still another disclosure of the present application relates to a medium comprising active vitamin D (calcitriol) or analogs thereof for use in improving fertilization of an egg.

Yet a disclosure of the present application relates to a composition comprising active vitamin D or analogs for use in the treatment of infertility in a male or female subject. In here is also disclosed that said composition is to be administered to the female reproductive tract, such as the uterus. In another disclosure of the application, said composition further comprises spermatozoa or one or more fertilized eggs.

### Brief description of the figures

Figure 1
   Vitamin D and intracellular calcium in human spermatozoa. **A:** Corresponding changes in intracellular calcium after addition of 1 nM 1,25(OH)2D3 and 10 µM progesterone, n=7. **B:** Effect of 1β,25(OH)2D3, 1,25(OH)2D3 and 10 µM progesterone, n=7. Each trace represents the recorded calcium level in a single spermatozoa.
Figure 2
   Vitamin D and intracellular calcium in human spermatozoa. **A:** Spermatozoa pretreated with 10 µM nifedipine before treatment with 1,25(OH)2D3 (1 nM in all experiments) and 10 µM progesterone, n=20 **B:** Addition of 4 µM thapsigargin resulted in a slow increase in calcium, and 1,25(OH)2D3 was added after 9 minutes exposure to thapsigargin, n=8. **C:** Spermatozoa pretreated with 2 µM PLC-inhibitor U73122 and the corresponding changes in intracellular calcium after addition of 1,25(OH)2D3, n=13. **D:** Effect of 1,25(OH)2D3 in calcium free media without EGTA, n=15. **E:** Effect of 1,25(OH)2D3, progesterone and ionomycin in calcium free media with 5 µM EGTA, n=14 **F:** Effect of 1,25(OH)2D3 in calcium free media with EGTA followed by addition of calcium, n=3 A-B: data presented as mean with s.e.m. C-F: Each trace represents calcium levels in single spermatozoa. Notice different scale in E and F.
Figure 3
   Active vitamin D induces sperm motility and the acrosome reaction. **A:** Dose-Response relationship between 1,25(OH)2D3 and sperm motility (ABC) under non-capacitating conditions. Data presented as mean and error bars indicate s.e.m. **B:** Motility assessment in 16 normal men with and without addition of 1 nM 1,25(OH)2D3, thick horizontal bars indicate mean. **C:** Acrosome reacted spermatozoa isolated after percoll gradient centrifugation treated with or without 1 nM 1,25(OH)2D3, under capacitating conditions. EqAR: percentage of spermatozoa with equatorial band following PSA staining. AR: percentage acrosome reacted spermatozoa following PSA staining. Data presented as mean and error bars represents 95% CI.
Figure 4

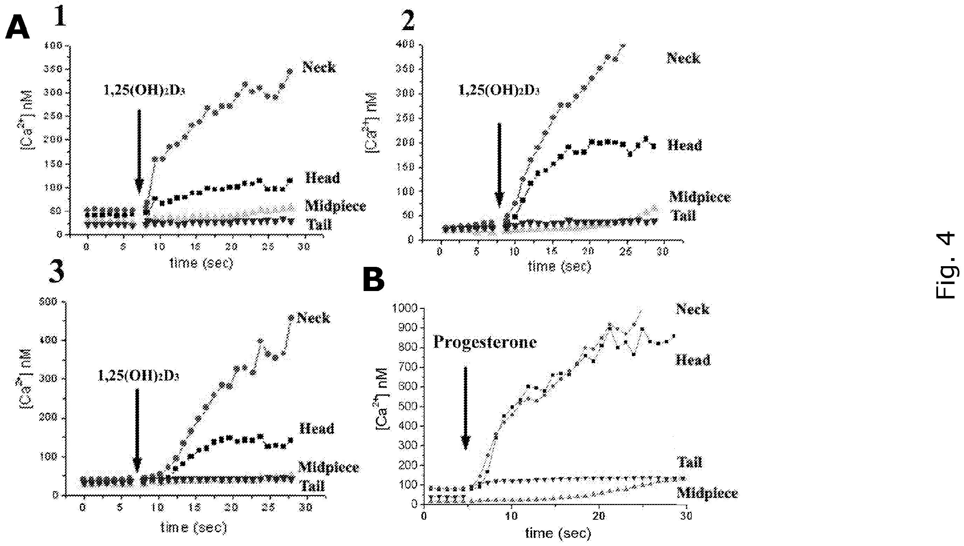

   Figure 4 shows spatio-temporal calcium release in human spermatozoa and induction of sperm motility. A1-A3: Spatio-temporal increase in calcium concentration [Ca²⁺]ᵢ in the head, neck, midpiece and principal piece of the tail following treatment with 1nM 1,25(OH)₂D₃. B: Mean spatio-temporal increase in [Ca²⁺]ᵢ following treatment with 1 µM progesterone
Figure 5

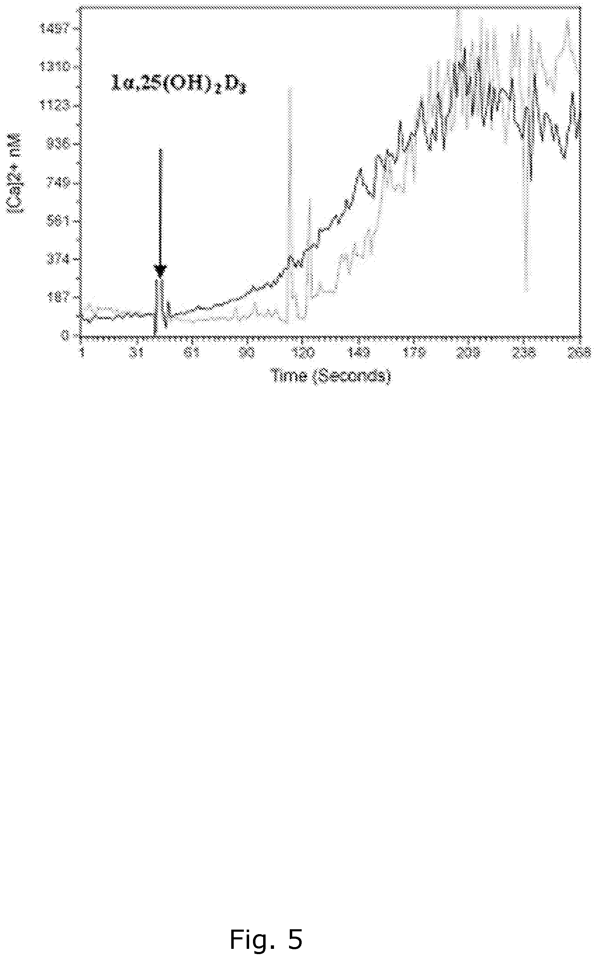

   Figure 5 shows vitamin D and intracellular calcium concentration [Ca²⁺]ᵢ in Fura 2 loaded human spermatozoa. A: Rapid change in intracellular calcium levels after addition of 1 nM 1,25(OH)₂D₃ despite pre-treatment with 100 nM ZK159222 each trace represents [Ca²⁺]ᵢ in single spermatozoa.
Figure 6

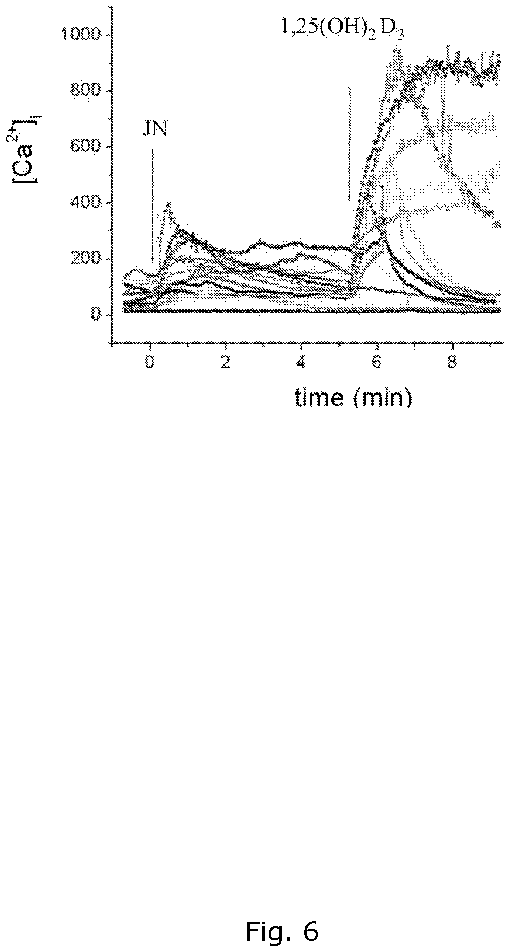

   Figure 6 shows rapid change in intracellular calcium concentration [Ca²⁺]ᵢ following treatment with 1 nM 1,25(OH)₂Lumisterol₃ (JN) and 1 nM 1,25(OH)₂D₃, each trace represents [Ca²⁺]ᵢ in single spermatozoa. Note different gradiations.
Figure 7

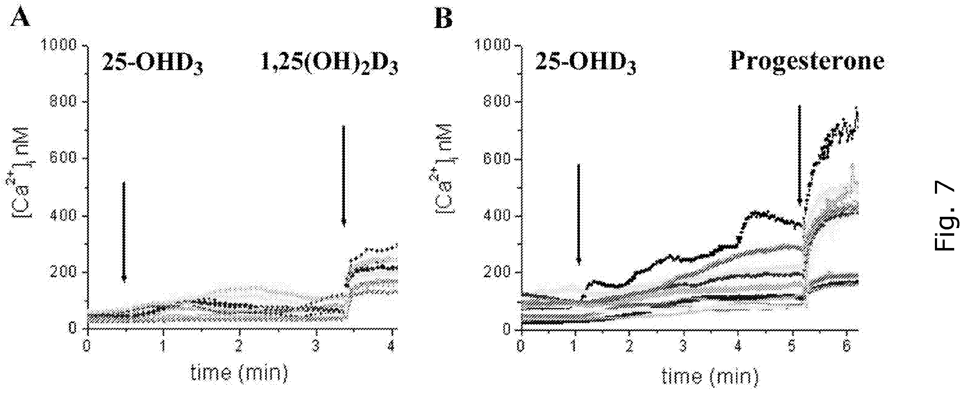

   Figure 7 shows vitamin D and intracellular calcium concentration [Ca^{2+]}ᵢ in Fura 2 loaded human spermatozoa. A: Corresponding changes in [Ca²⁺]ᵢ after addition of 1nM 25(OH)D₃ and 1 nM 1,25(OH)₂D₃. B: Increase in [Ca²⁺]ᵢ following treatment with 1 µM 25(OH)D₃ and 10 µM progesterone, each trace represents calcium levels in single spermatozoa.
Figure 8

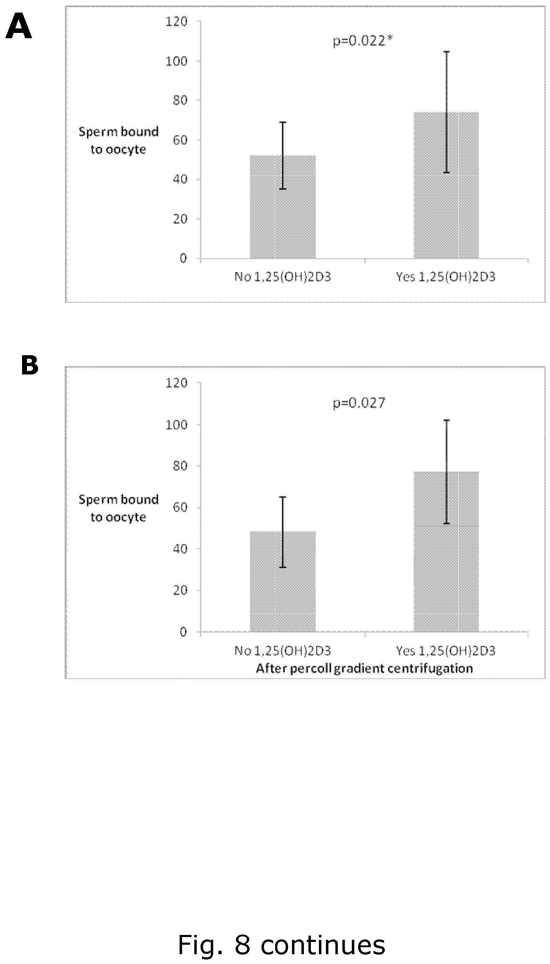

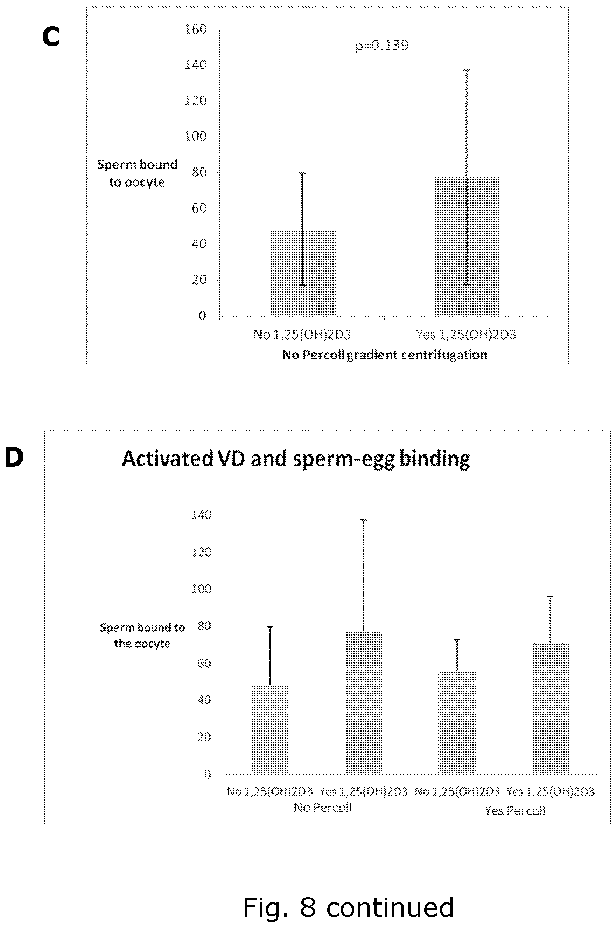

   Figure 8 shows sperm-zona pelucida binding test using 5 semen donors. We tested 60 oocytes (12 oocytes/patient), 30 in each group (+/- 1 nM activated vitamin D, 1,25(OH)2D3). Sperm-zona pelucida binding was tested using spermatozoa isolated with and without percoll gradient centrifugation (6 oocytes in both groups). **A:** Data presented as mean sperm egg binding error bars indicate 95% confidence intervals. **B:** Mean sperm-egg binding with 95% confidence intervals in spermatozoa isolated by percoll centrifugation. **C:** Mean sperm-egg binding with 95% confidence intervals in spermtatozoa that were not isolated by percoll gradient centrifugation. **D:** Mean sperm-egg binding stratified according to pretreatment. Error bars indicate S.D., (*) marks significant result p<0.05
Figure 9
   Vitamin D and sperm motility. **A:** mean sperm motility (ABC) with and without activated vitamin D (1,25(OH)2D3) stratified according to pretreatment with percoll gradient centrifugation. Error bars indicate 95% CI. **B:** mean progressive sperm motility (ABC) with and without activated vitamin D (1,25(OH)2D3) stratified according to pretreatment with percoll gradient centrifugation. Error bars indicate 95% CI. **C:** Sperm motility in spermatozoa either separated by percoll gradient centrifugation or with no Percoll centrifugation evaluated 1 hour (dark) and 24 hours (bright) after treatment with 1 nM 1,25(OH)2D3 at room temperature. **D:** Sperm motility in spermatozoa either separated by percoll gradient centrifugation or with no Percoll centrifugation evaluated 1 hour (dark) and 24 hours (bright) after treatment with vehicle at room temperature. The present invention will now be described in more detail in the following.
Figure 10
   Sperm-zona pelucida binding test. 16 oocytes, 4 in each group (+/- 10 nM of the vitamin D analog JN). Sperm-zona pelucida binding was tested using spermatozoa isolated with and without percoll gradient centrifugation. Data presented as mean sperm egg binding to four oocytes.
Figure 11
   Sperm motility assessed after 1 hour (dark) and 24 hours (white) using the vitamin D analog JN.

### Detailed description of the invention

### Method for improving the ability of a spermatozoa to fertilize an egg in vitro and/or vivo

It may be advantageous to be able to improve the fertilization of an egg in vitro or in vivo. Thus, a disclosure of the present application relates to a method for improving the ability of a spermatozoa to fertilize an egg in vitro and/or vivo, said method comprising
a) providing a medium comprising active vitamin D (calcitriol) or analogs thereof,
b) providing a sample of spermatozoa, and
c) incubating said sample of spermatozoa with said medium.

Thus, an aspect of the present invention relates to a method for improving the ability of a spermatozoa to fertilize an egg in vitro, said method comprising
- providing a medium comprising active vitamin D (calcitriol) or analogs thereof,
- providing a sample of spermatozoa, and
- incubating said sample of spermatozoa with said medium.
wherein said improved ability of a spermatozoa to fertilize an egg is at least partly due to an increased binding of spermatozoa to the egg;
wherein said analogs are agonists which can bind and release the non-genomic effect via VDR (Vitamin D receptor) in spermatozoa OR wherein said analogs are selected from the group consisting of the list of table 1 and 1,25(OH)₂ Lumisterol₃ (JN) and 25-OHD₃;
wherein said active vitamin D or analogs thereof is present at a concentration of 10⁻¹⁵- 10⁻⁴ M.

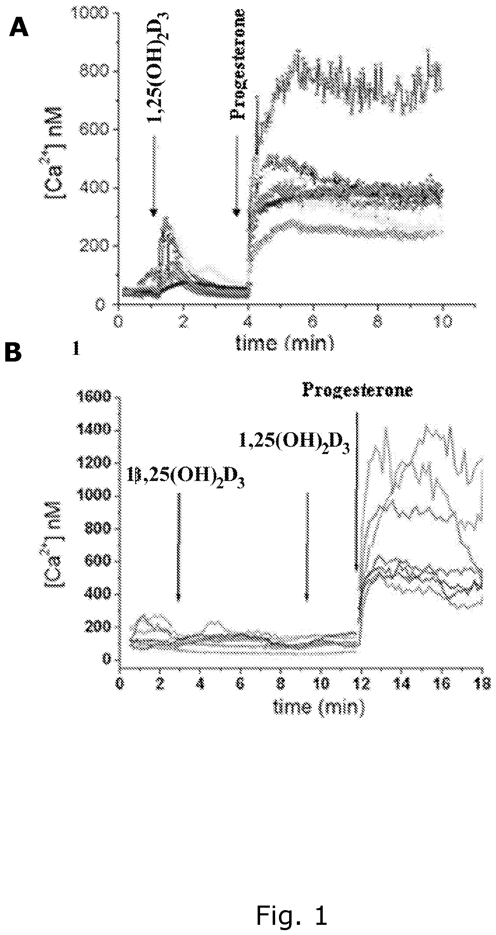

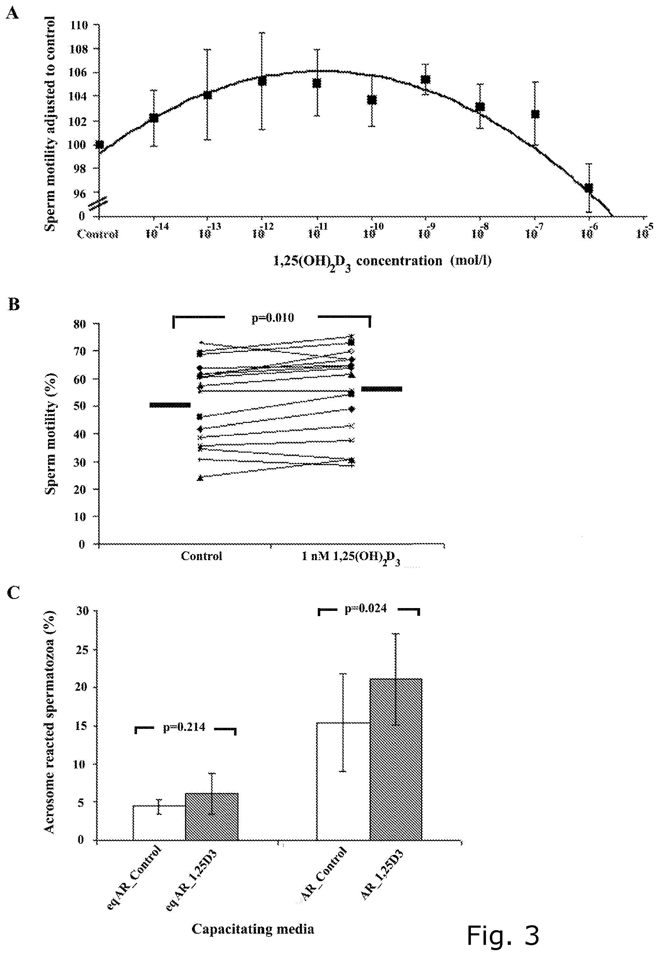

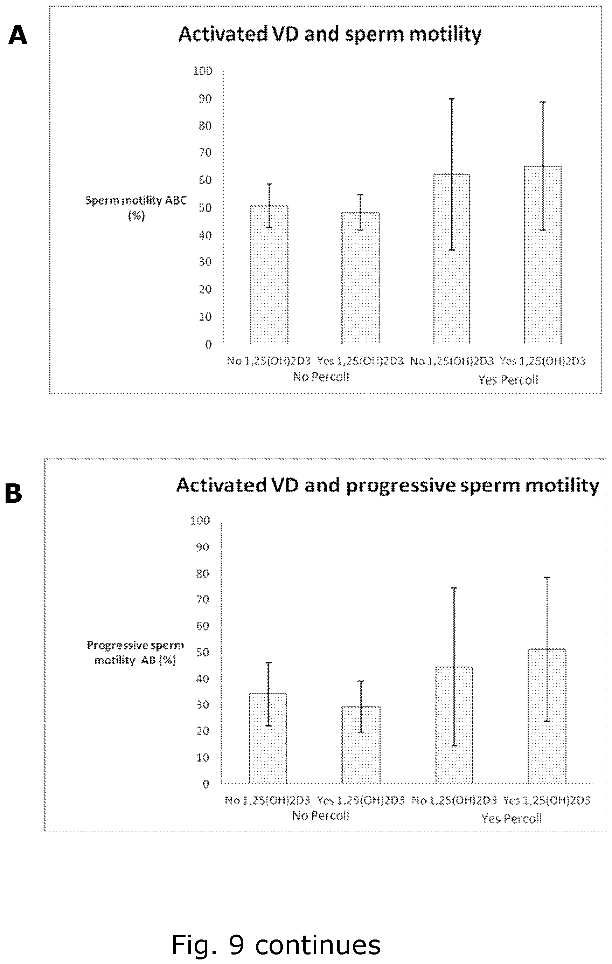

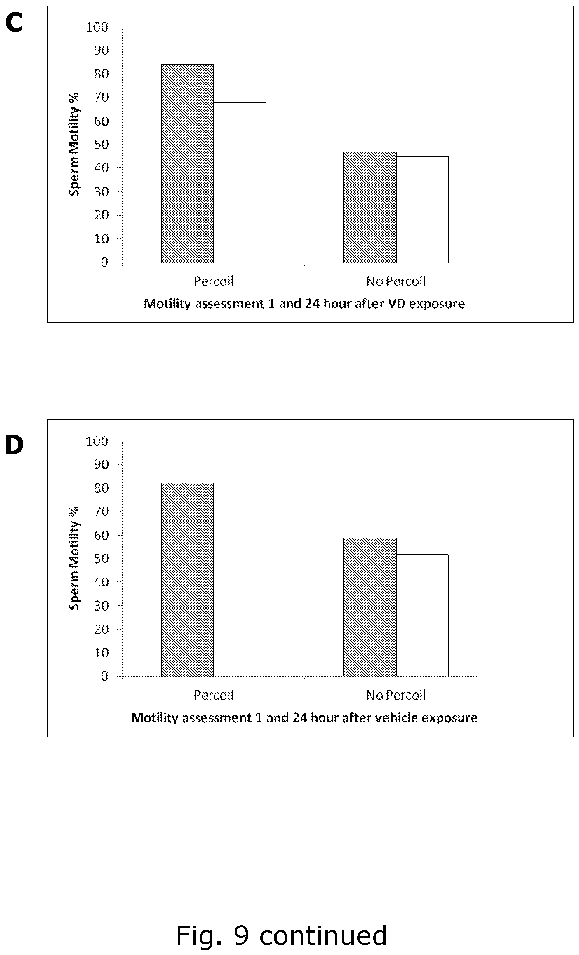

The inventors have established a clear connection between the presence of active vitamin D in a medium and a transient calcium release in the neck of the spermatozoa. Active vitamin D is the first identified substance which is able to induce a transient calcium release from the intracellular calcium storage in the neck. Other substances such as progesterone or calcium, ionophores such as ionomycin and others induce a calcium increase from multiple calcium storages such as (the neck, pricipial piece of the tail, acrosome and mitochondria) and induce an irreversible or at least prolonged calcium increase (figure 4D). This induces the acrosome reaction and most spermatozoa become immotile after usage of calcium ionophores due to the prolonged high calcium concentration. The vitamin D response differs, because there is no calcium increase in the tail, and the increase initiates in the neck and propagates up in the head. Activated vitamin D or active derivatives thereof induce a short increase in calcium concentration, which induces sperm motility and the acrosome reaction simultaneously, which is beneficial for IVF because the changes in the spermatozoa mimics the capacitation process happening in the female reproductive tract. Taken together, a number of the discovered effects of active vitamin D or analogs thereof which have been identified are:
a) Induction of a transient calcium release in the neck of the spermatozoa that propagates up in the head (figure 1A and 4B-1-3).
b) Induction of the acrosome reaction in capacitating media due to the propagating calcium increase in the head simultaneously with the transient calcium release (figure 3C).
c) The spermatozoa are motile for a longer period of time compared to control treated sperm (figure 3AB + figure 9).
d) An improved egg-sperm binding, which cannot solely be attributed to the increased motility of the spermatozoa (example 5 + Figures 8 and 9)
e) Activated vitamin D may assist in selecting high quality gametes for fertilization since VDR predominantly is expressed in high quality spermatozoa.

Thus, a common inventive concept of active vitamin D or analogs thereof according to the present invention may be the provision of compounds which elicit the above effects.

In an embodiment, the medium further comprises Ca²⁺ e.g. in a concentration in the range 10⁻⁹ - 10⁻² M and/or bicarbonate e.g. in the range 1-40 mM and/or BSA.

In another embodiment, transient calcium release is to be understood as calcium levels restore back to baseline levels within 5 minutes, such as within 4 minutes, such as within 3 minutes, such as within 2 minutes, such as within 90 seconds or such as within 60 seconds.

In a further embodiment, the spermatozoa are motile 45 minutes after treatment with active vitamin D or derivatives thereof, such as motile after at least 1 hour, such as at least 5 hours such as at least 10 hours, such as at least 15 hours, such as at least 20 hours. Example 5 + figure 9 show that the spermatozoa may be motile for at least 24 hours after treatment with active vitamin D.

In an embodiment, according to the invention the activity of active vitamin D or analogs thereof do not initially induce substantial calcium release in other parts of the spermatozoa (e.g. the tale) than the neck that subsequently propagates up in the head and occasionally down the midpiece. Thus, no substantial calcium release is detectable in e.g. the tail (Figure 4).

A number of terms for active vitamin D may be used such as calcitriol, 1,25-dihydroxycholecalciferol, 1,25-dihydroxyvitamin D3, 1,25-(OH)2D3, VD or 1,25(OH)2D). Calcitriol is the hormonally active form of vitamin D with three hydroxyl groups. CAS number 32222-06-3. The different terms may be used throughout this text.

### Analogs

A large number of calcitriol analogs exist with different binding properties and thus potency compared to calcitriol. In an embodiment, said analogs of active vitamin D are selected from the group listed in Table 1. However, the person skilled in the art may be able to identify others analogs, which may also form part of the present invention. US 2004116393 (A1) discloses a long list of vitamin D analogs. Analogs may also be defined as agonists which can bind and release the non-genomic effect via VDR and which comprises a cholesterol component. Analogs may also be defined as agonists which can bind and release the non-genomic effect via VDR. In a further embodiment, analogs may also be defined as agonists which can bind and release the non-genomic effect via the VDR-ap pocket.

Examples of analogs are listed in table 1.

**TABLE 1**

| **Substance** | **CAS Registry & name** |
|---|---|
| calcipotriene | 112965-21-6 9,10-Secochola-5,7,10(19),22-tetraene-1,3,24-triol, 24-cyclopropyl-, (1alpha,3beta,5Z,7E,22E) |
| maxacalcitol | 103909-75-7 |
| seocalcitol | 134404-52-7 |
| KH 1060 | 131875-08-6 1,3-Cyclohexanediol, 5-((1-(1-((4-ethyl-4-hydroxyhexyl)oxy)ethyl)octahydro-7a-methyl-4H-inden-4-ylidene)ethylidene)-4-methylene-, (1S-(1alpha(S*),3abeta,4E(1S*,3R*,5Z),7aalpha)) |
| 26, 26, 26, 27, 27, 27-hexafluoro-1,25-dihydroxyvitamin D3 | 83805-11-2 9,10-Secocholesta-5,7,10(19)-triene-1,3,25-triol, 26,26,26,27,27,27-hexafluoro-, (1alpha,3beta,5Z,7E) |
| 2-(3-hydroxypropoxy)-1,25-dihydroxyvitamin D3 | 104121-92-8 9,10-Secocholesta-5,7,10(19)-triene-1,3,25-triol, 2-(3-hydroxypropoxy)-, (1alpha,2beta,3beta,5Z,7E) |
| Ro 23-7553 | 118694-43-2 |
| 1,25-dihydroxyvitamin D3-26,23-lactone | 81203-50-1 |
| CB 1093 | 0 |
| 1,25-dihydroxy-19-norvitamin D3 | 130447-37-9 |
| TEI 9647 | 0 |
| 24,24-difluoro-1,25-dihydroxyvitamin D3 | 72696-49-2 |
| Ro 24-5531 | 0 |
| ZK159222 | 0 |
| calcitroic acid | 71204-89-2 24-Nor-9,10-secochola-5,7,10(19)-trien-23-oic acid, 1,3-dihydroxy-, (1alpha,3beta,5Z,7E) |
| 1,25-dihydroxy-16-ene-vitamin D3 | 124409-58-1 |
| BXL628 | 0 |
| 26, 26, 26, 27, 27, 27-hexafluoro-1,23,25-trihydroxyvitamin D3 | 114489-80-4 |
| 1,25-dihydroxy-previtamin D(3) | 57102-09-7 9,10-secocholesta-5(10),6,8-triene-1,3,25-triol, (1 alpha,3 beta,6Z) |
| 1,25-dihydroxy-24-oxo-vitamin D3 | 76338-50-6 9,10-Secocholesta-5,7,10(19)-trien-24-one, 1,3,25-trihydroxy-, (1alpha,3beta,5Z,7E) |
| 1,25-dihydroxy-21-(3-hydroxy-3-methylbutyl)vitamin D(3) | 0 |
| 1,23,25-trihydroxyvitamin D3 | 86701-33-9 9,10-Secocholesta-5,7,10(19)-triene-1,3,23,25-tetrol, (1alpha,3beta,5Z,7E,23S) |
| carcinomedin | 108387-51-5 |
| 1,25-dihydroxy-24-oxo-16-ene-vitamin D3 | 0 |
| 1,25-dihydroxyvitamin D3-3-bromoacetate | 0 |
| JK 1624F2-1 | 0 |
| 24-fluoro-1,25-dihydroxycholecalciferol | 86677-62-5 |
| 22-dehydro-1,25-dihydroxy-24-homovitamin D3 | 103732-08-7 |
| EB 1213 | 167357-73-5 |
| QW 1624F2-1 | 0 |
| 1,25(OH)2-16-ene-23-yne-26,27-hexafluoro-19-nor-D3 | 0 |
| ZK 168281 | 0 |
| 1,25-dihydroxycholecalciferol glycoside | 0 |
| 1,25-dihydroxy-26,27-dimethylcholecalciferol | 97473-92-2 |
| 1,25-dihydroxyvitamin D3-3-(N-(4-azido-2-nitrophenyl)glycinate) | 98728-28-0 |
| CB 966 | 128312-71-0 1,3-Cyclohexanediol, 5-001-(((6-ethyl-6-hydroxy-1-methyloctyl)octahydro-7a-methyl-4H-inden-4-ylidene)ethylidene)-4-methylene-, 01R-01alpha(R*),3abeta,4E(1R*,3S*,5Z),7aalpha |
| GS 1500 | 0 |
| 2-methylene-1,25-dihydroxy-19-norvitamin D3 | 0 |
| ZK 191784 | 0 |
| 1,25-dihydroxy-23-oxo-vitamin D3 | 82095-23-6 9,10-Secocholesta-5,7,10(19)-trien-23-one, 1,3,25-trihydroxy-, (1alpha,3beta,5Z,7E) |
| 1,25-dihydroxyvitamin D3 monoglucuronide | 82694-73-3 beta-D-Glucopyranosiduronic acid, (1alpha,3beta,5Z,7E)-dihydroxy-9,10-secocholesta-5,7,10(19)-trienyl |
| 24-homo-1,25-dihydroxyvitamin D3 | 103656-40-2 |
| 26-homo-1,25-dihydroxyvitamin D3 | 105687-81-8 |
| 26,27-diethyl-1,25-dihydroxyvitamin D3 | 0 |
| 24,24-difluoro-24-homo-1,25-dihydroxyvitamin D3 | 115540-42-6 |
| 24,24-dihomo-1,25-dihydroxycholecalciferol | 114694-09-6 1,3-Cyclohexanediol, 4-methylene-5-((octahydro-1-(7-hydroxy-1,7-dimethyloctyl)-7a-methyl-4H-inden-4-ylidene)ethylidene)-, (1R-(1alpha(R*),3abeta,4E(1R*,3S*,5Z),7aalpha)) |
| 1,25-dihydroxy-23-thiavitamin D3 | 87480-00-0 |
| 22-dehydro-1,25-dihydroxy-24-dihomovitamin D3 | 154356-84-0 |
| DD 003 | 107793-48-6 Nickel alloy, base, Ni,Al,Co,Cr,Mo,Ti,W (DD3) |
| MC 1301 | 134523-85-6 |
| 25-O-ethyl-calcitriol | 0 |
| 1,25-dihydroxy-18-norvitamin D3 | 0 |
| KH 1266 | 0 |
| ZK 161422 | 0 |
| 2-chloro-1-epicalcitriol | 0 |
| 1-hydroxy-19-norvitamin D3 | 0 |
| 2,2-dimethyl-1,25-dihydroxyvitamin D3 | 0 |
| Ro43 83582 | 0 |
| 24-keto-1,23,25-trihydroxyvitamin D3 | 87678-01-1 |
| 1,23-dihydroxy-24, 25, 26, 27-tetranorvitamin D3 | 97903-37-2 |
| Alfacalcidol | (1,hydroxyvitamin D3) |
| Paricalcitiol | |
| 25-hydroxy-vitamin D | |
| ZK1916784 | |
| 0MC 903 | 1a ,24S-(OH),-22-ene-26,27-cyclopropyvl Vitamin D |
| MC 1288 | 20-epi-1a,25-dihydroxyvitamin D |
| 22-oxacalcitriol | |

Other analogs which form part of the present invention are the 6-cis-locked agonist 1,25(OH)₂ Lumisterol₃ (JN) and 25-OHD₃. Thus, in an embodiment, the analogs are selected from the list of table 1 and 1,25(OH)₂ Lumisterol₃ (JN) and 25-OHD₃. The transient calcium release induced in spermatozoa by JN and 25-OHD₃ are shown in example 4.

It is to be understood that the analogs according to the invention provide the same effects as active vitamin D described above, though the specific activity may vary depending on the affinity for the vitamin D receptor.

### Concentration of active vitamin D or analogs thereof

Active vitamin D or analogs thereof may be present in the medium at different concentrations to have the optimal effect. Thus, in an embodiment, said active vitamin D or analogs thereof is present at a concentration of 10⁻¹⁵ - 10⁻⁴ M, such as 10⁻¹⁴- 10⁻⁸ M such as 10⁻¹²- 10⁻⁹ such as 10⁻¹¹- 10⁻⁹ or such as 10⁻¹⁰- 10⁻⁹. The most promising effect is seen in the interval 10⁻¹⁴- 10⁻⁸ M, however other concentrations may be preferred for some of the analogs.

### Purification of spermatozoa sample

The sample of spermatozoa is preferably a purified sample, which is a standard procedure, however in some instances such purification may not be necessary. In an embodiment, said spermatozoa sample is a purified sample. All methods used for separation of human spermatozoa from seminal plasma to yield a final preparation containing a high percentage of morphologically normal and motile cells, free from debris, non-germ cells and dead spermatozoa, is important for clinical practice. Examples of such methods are density-gradient-centrifugation, spin-up, glass-wool columns, simple centrifugation, percoll gradient, and simple washing. All such purification procedures are known to the skilled person. In an embodiment the purified sample comprises motile spermatozoa.

It may be advantageous if the sample comprises a minimum number of spermatozoa to improve the chance for fertilization according to the aspects of the present invention. Thus, in an embodiment, the concentration of spermatozoa in the sample is at least 100,000 /ml, such as at least 500,000 /ml, such as at least 800,000 /ml, such as at least 1 million /ml, such as at least 5 million /ml, such as at least 10 million /ml, such as at least 15 million /ml, or such as at least 20 million /ml, concentration 63 million/ml.

It may also be advantageous if the sample comprises a minimum number of motile spermatozoa to improve the chance for fertilization according to the aspects of the present invention, since the spermatozoa have to reach the egg. Thus, in an embodiment, the number of motile spermatozoa in the sample is in the range concentration of spermatozoa in the sample is at least 5%, such as at least 10%, such as at least 20%, such as at least 30%, such as at least 40%, such as at least 50%, or such as at least 60%.

The person skilled in the art knows how to determine the amount of spermatozoa in a sample, similar he is well aware of how to calculate the percentage of motile spermatozoa. See e.g. the example section for further information.

### In vitro fertilization

Different types of in vitro fertilizations form part of the present invention. In an embodiment, said in vitro fertilization is Intracytoplasmic sperm injection (ICSI) or in vitro fertilization (IVF). Other types of in vitro fertilizations may be known to the skilled person.

### In vivo fertilization

Different types of in vivo fertilizations is disclosed in here. In here is disclosed that a said in vivo fertilization is gamete intrafallopian transfer or intrauterine insemination (IUI).

### Evaluation

Before a sample of spermatozoa is incubated with an egg or during incubation, it may be advantageous to evaluate the state of the spermatozoa. Thus, in an embodiment, the method according to the invention further comprises evaluating whether the spermatozoa have undergone capacitation, hyperactivation or the acrosome reaction. To decide which ART method is suitable for treating an infertile couple, routine semen analysis may be conducted to determine the number of spermatozoa, their motility and the percentage with normal morphology. Such analysis can be supported by test of motility induction in capacitating media, induction of hyperactivation and the acrosome reaction, which all are needed before fertilization of the egg can be achieved. Capacitation is an event happening when the extracellular component changes with alters the signaling in the spermatozoa enabling fertilization. One of these events is hyperactivation, which is characterized by a rapid forward movement of spermatozoa needed to penetrate the surrounding cumulus layer of the oocyte and achieve sperm-egg binding. The acrosome reaction is normally induced by sperm egg binding, which allows the spermatozoa to penetrate the egg, although it can also be induced by progesterone and ionophores. Capacitation, hyperactivation and the acrosome reaction are all dependent on a calcium concentration increase in the spermatozoa. The intracellular calcium storage is thought to be involved in all three processes and it is here shown that activated VD is regulating calcium release from this storage and may thus be implicated in both capacitation, hyperactivation and the acrosome reaction. By testing the effect of activated vitamin D you may be able to predict the fertility chances and decide upon the suitable ART method.

In a more specific embodiment, said evaluation comprises measuring transient calcium release in the neck of the spermatozoa. Instead of testing activated vitamin D on capacitation, hyperactivation and the acrosome reaction you may investigate whether it is able to induce a transient calcium increase. If so, this is indicative of an effect on capacitation, hyperactivation and the acrosome reaction, which may help in the selecting the ART method or guiding the infertile couple.

### Origin of egg and spermatozoa

The spermatozoa and eggs of the present disclosure are preferably of mammalian origin. Thus, in the disclosure, said egg and/or spermatozoa are of mammalian origin, such as human, horse, pig, cow or sheep. More preferably, the egg and spermatozoa are of identical species. The egg and the spermatozoa are of identical species when they are of human origin.

### Method for improving in vitro fertilization of an egg

When working with in vitro fertilization the egg is fertilized outside the body of the female. Thus, invention disclosure of the present application relates to a method for improving in vitro fertilization of an egg, said method comprising
- providing a medium comprising active vitamin D (calcitriol) or analogs thereof,
- providing a sample of spermatozoa,
- providing a sample comprising one or more eggs,
- incubating said sample of spermatozoa and said sample of one or more human eggs in said medium.

The order of incubation may vary. Thus, in here is disclosed that the eggs and spermatozoa are firstly incubated in a medium comprising calcitriol or analogs thereof before being incubated together. In here is also disclosed that one or more eggs is incubated with the medium for a period of time before the spermatozoa are added. In a further disclosure, the spermatozoa are incubated in the medium for a period of time before the one or more eggs are added.

### Use of a medium comprising active vitamin D or analogs thereof

A medium comprising calcitriol are beneficial for the fertilization of an egg, e.g. by promoting transient calcium release in the neck of spermatozoa and thereby improve sperm-egg binding. Thus, disclosed in here is the use of a medium comprising active vitamin D (calcitriol) or analogs thereof, for improving in vivo or in vitro fertilization of an egg.

Similar, yet a disclosure of the present application, relates to the use of a medium comprising active vitamin D (calcitriol) or analogs thereof, for improving the ability of spermatozoa to fertilize an egg in vivo or in vitro.

In a more specific embodiment, the improved or in vitro fertilization is caused by improved sperm-egg binding. Similar, in an embodiment the use for improving the ability of spermatozoa to fertilize an egg in vivo or in vitro is caused by improved sperm-egg binding.

### Medium comprising active vitamin D or analogs thereof for use

In yet a further disclosure, the application relates to a medium comprising active vitamin D (calcitriol) or analogs thereof for use in the treatment of infertility in a male or female subject. In another disclosure, the application relates to a medium comprising active vitamin D (calcitriol) or analogs thereof for use in improving fertilization of an egg. Again, in an embodiment the improvement relates to improved egg-sperm binding.

Thus, an aspect of the invention relates to the use of a composition comprising active vitamin D (calcitriol) or analogs thereof for, in vitro, improving the ability of a spermatozoa to bind to an egg;
wherein said analogs are agonists which can bind and release the non-genomic effect via VDR (Vitamin D receptor) in spermatozoa OR wherein said analogs are selected from the group consisting of the list of table 1 and 1,25(OH)₂ Lumisterol₃ (JN) and 25-OHD₃;
wherein said active vitamin D or analogs thereof is present at a concentration of 10⁻¹⁵ - 10⁻⁴ M.

### Egg-sperm binding

To evaluate the direct effect of active vitamin D and analogs thereof on direct fertilization of an egg, motility tests are not sufficient, since increased motility does not necessarily induce increased fertilization. For example, male subjects exist which have normal motility of their spermatozoa, but the spermatozoas are still unable or impaired in fertilizing an egg. A typical example is a male person having spermatozoa with round heads (globozoospermia). Total globozoospermia is diagnosed by the presence of 100% round-headed spermatozoa lacking an acrosome and such spermatozoa are motile, but unable to fertilize the egg Thus, more specific means for increasing fertilizing may be required.

Thus, in an embodiment the increased fertilization of an egg is at least partly due to an increased binding of spermatozoa to the egg. In another embodiment, the increased fertilization of an egg is due to an increased binding of spermatozoa to the egg. In yet an embodiment, the increased fertilization is caused by induction of the acrosome reaction before, during or after binding to the egg. The acrosome reaction facilitates penetration of the surrounding cumulous cells and the fertilization of the oocyte. When using e.g. intracytoplasmic sperm injection (ICSI) for IVF, the implantation rate is higher in oocytes injected with spermatozoa that have undergone acrosome reaction (∼40%) vs. those injected with nonreacted spermatozoa (∼10%). The acrosome reaction may be stimulated in vitro by substances such as progesterone. However, as described in example 1, progesterone does not induce a transient calcium release which is the case for active vitamin D or analogs thereof and the effect is therefore different.

Though several compounds such as progesterone, coffeine, cAMP, cGMP, PDEI have the potential to increase sperm motility, such an increase is normally only temporary and may be dramatically reduced after a short period (e.g. 30 minutes). The increased sperm motility induced by active vitamin D or analogs thereof is different since it is persistent after 60 minutes and the mechanism initiating (calcium release in the neck) is exclusive for vitamin D since all other compounds mediate calcium release by other downstream mechanisms, which often require calcium influx from the extracellular compartment.

In an additional embodiment, the increase in egg-sperm binding is in the range 10-200% compared to untreated samples, such as in the range 20-200%, such as in the range 30-200%, such as in the range 40-200%, such as in the range 10-150%, such as in the range 10-100% or such as in the range 10-80%. Example 5 provides examples of such calculation methods.

### Composition

Active vitamin D (calcitriol) may be used in the treatment of infertility in a male or female subject. Thus, in a further disclosure, the application relates to a composition comprising active vitamin D or analogs thereof for use in the treatment of infertility in a male or female subject, e.g. in a concentration of 10⁻¹⁵ - 10⁻⁴ M. The treatment may be considered to treat either of the male or female (or both) since poor semen quality affect the male directly and therefore also the fertilization of the egg. In a disclosure, the increased fertilization of an egg is at least partly due to an increased binding of spermatozoa to the egg. Thus, in yet a disclosure the application relates to a composition comprising active vitamin D or analogs thereof for improving sperm-egg binding in vivo or in vitro. The composition may further comprise a pharmaceutically acceptable carrier. In the present case the pharmaceutical is active vitamin D or analogs thereof for improving fertilization of an egg, e.g. through improved sperm-egg binding.

Infertility may be defined in different ways.

Infertility in a couple may be defined as 12 months wish to conceive with no success. Clinical assessment of male infertility/subfertility is primarily based on the analysis of semen quality. The most informative parameters are sperm concentration, total sperm count, sperm motility and morphology. Reference levels have been debated and were recently changed (WHO 2010). Female infertility may be evaluated by investigation of all the female reproductive organs and endocrine tests. Common causes of infertility of females include: ovarian failure (maturation or ovulation problems), tubal blockage, Uterine problems, endocrine problems, systemic disease and age-related factors.

### Administration route

The composition disclosed in here may be administered directly in the female reproductive tract, e.g. in the form of a fertility lubricant. Thus, in here is disclosed that said composition may be administered to the female reproductive tract, such as the uterus. By administering such composition directly to the female reproductive tract e.g. before sexual intercourse, the effect seen on the semen ex vivo will likely also take place in vivo and therefore improve fertilization. Thus, in here is a disclosed that the increased fertilization of an egg is at least partly due to an increased binding of spermatozoa to the egg. In here is further disclosed that the composition further comprises a lubricant such as one or more water-based lubricants such as cellulose, carrageenan, polyethylene glycol or glycerin solutions. Oil-based lubricant may also be used such as petroleum based or based on plant oils. Lubricants for sexual purposes are well known in the art.

Thus, in yet a disclosure the application relates to a composition comprising active vitamin D or analogs thereof for improving sperm-egg binding, wherein said composition is administered in the female reproductive tract. In here is also disclosed that said composition may be administered to the female reproductive tract, before, during or after sexual intercourse or before, during or after in vivo fertilization techniques as described above. The above example also covers the peculiar case where male infertility is treated by administration of the composition according to the invention to the female reproductive tract.

In here is also disclosed that administration before sexual intercause or before in vivo fertilization techniques as described above refers to a period of 24 hours to 5 seconds, such as 12 hours to 5 seconds, such as 8 hours to 5 seconds, such as 4 hours to 5 seconds, such as 2 hours to 5 seconds, such as 1 hour to 5 seconds, such as 30 minutes to 5 seconds such as 15 minutes to 5 seconds, or such as 5 minutes to 5 seconds. In yet a disclosure, it is disclosed that administration after sexual intercourse or after in vivo fertilization techniques as described above refers to a period of 5 seconds to 24 hours, such as 5 seconds to 12 hours, such as 5 seconds to 8 hours, such as 5 seconds to 4 hours, such as 5 seconds to 2 hours, such as 5 seconds to 1 hour, such as 5 seconds to 30 minutes such as 5 seconds to 15 minutes, or such as 5 seconds to 5 minutes.

Fertility lubricants, also known as sperm-friendly lubricants, are formulated to be safe for use by couples who are trying to conceive. Fertility lubricants are pH and electrolyte-balanced and have a specific osmolality range that is safe for sperm. In addition to having the correct pH and osmolality range, fertility lubricants should be free of chemicals that can harm sperm. Fertility lubricants are non-spermicidal and do not harm viability or motility of human sperm.

A fertility lubricant that contains calcium (Ca²⁺) and magnesium (Mg²⁺) ions and has an optimal pH and osmolality range better mimics the natural fertile cervical fluids to alleviate the problem of vaginal dryness and to assist couples trying to conceive a baby naturally. Thus, in here is also disclosed that the composition further comprises Ca²⁺ and/or Mg²⁺, such as in the range (10⁻⁹ - 10⁻² M). When an egg has been fertilized in vitro it must be inserted in the female. Similar if spermatozoa have been activated/stimulated in a medium as disclosed in the present invention it may be inserted in the female for in vivo fertilization. Thus, in here is also disclosed that said composition further comprises spermatozoa or one or more fertilized eggs. Again such compositions are to be seen as improving infertility in both the male and the female.

It should be noted that embodiments and features described in the context of one of the aspects of the present invention also apply to the other aspects of the invention.

The invention will now be described in further details in the following non-limiting examples.

### Examples

### Material and methods

### Cross-sectional association study

From an ongoing monitoring study of semen quality of young men from the general Danish population (Jørgensen et al., 2010A; Jørgensen et al., 2010B), 308 men participating January to December 2007 were retrospectively investigated for an association between serum VD levels and semen quality. Eight were excluded due to use of anabolic steroids (N=3), failure to deliver a semen sample (N=2), obstructive azoospermia (N=1), lack of blood sampling (N=1) and Gilbert Meulengracht disease (N=1), leaving 300 men in the study. The men delivered one semen sample, one blood sample (stored at - 20°C until analysis), had a physical examination performed and answered a comprehensive questionnaire. The questionnaire included information on age, previous or current diseases, any known history of fertility, medication, etc.

### Assessment of vitamin D, reproductive hormones and other biochemical variables

Measurements of 25-OH vitamin D rely on determination of both 25-OH Vitamin D2 and D3 and were conducted by isotope dilution liquid chromatography tandem mass spectrometry (LC-MS/MS). The method was tested by the use of 25-OH Vitamin D2 (MW 412.7, Fluka No. 17937), 25-OH Vitamin D3 (MW 400.7 Sigma-Aldrich No. H 4014) and an internal standard [2H6]-25-OH Vitamin D3 (26,26,26,27,27,27-hexadeutero-25-hydroxycholecalciferol, Mw: 406.7 Synthetica, Norway) dissolved in 50% ethanol/acetic acid, 200 mmol/l, pH 4.0. Vitamin D standard (NIST, USA, No. SRM 972) was used as primary calibrator, while working calibrators were 25-OH Vitamin D2 and D3 added to human serum to obtain four calibrators with D2 concentration up to 200 nmol/l, and D3 concentration up to 300 nmol/l. 25-OH Vitamin D3 and D2 were analyzed after liquid-liquid extraction. Internal standard (300 µl) was added to serum, control or calibrator (300 µl), and vortex-mixed for 2 minutes with 1 ml n-heptane. After mixing, the heptane phase was transferred to a heating block, and evaporated to dryness at 75°C under a stream of nitrogen. The residue was dissolved in 300 µl MeOH:H2O (80:20), and applied to the LC-MS/MS instrument (sample vol. 50 µl). The LC-MS/MS system consisted of Waters Alliance 2795 HPLC interfaced to Waters Micromass Quattro Micro API tandem quadrupole mass spectrometer (Waters, Milford, MA). Chromatographic separation was achieved with a Waters analytical column (Atlantis dC18, 3 µm, 2.1 x 50 mm, part no. 186001291) equilibrated with MeOH:H2O (80:20) containing 0.1% formic acid. Applying a linear gradient of 100% MeOH containing 0.1% formic acid over 3 minutes eluted the VD metabolites. Flow rate was 0.4 ml/min, and total cycle time on LC-MS/MS was 8 minutes. The tandem mass spectrometer used positive electrospray ionization at an operating voltage of 3.5 kV, and a desolvation temperature of 350°C. The instrument was operated in MRM (multiple-reaction monitoring) mode, with the following transitions: m/z+ 413.15/395.2 for 25-OH VD2, m/z+ 401.15/383.15 for 25-OH VD3, and m/z+ 407.2/389.3 for [2H6]-25-OH VD3. The inter-assay coefficients of variation (CV) for 25-OH VD3 were 2.2% and 2.8% at 30 and 180 nmol/l, respectively, and for 25-OH VD2 were 7.6% and 4.6 % at 43 and 150 nmol/l, respectively.

Serum FSH levels were determined using a time-resolved immunofluorometric assay (Delfia; Wallac, Turku, Finland) and Inhibin-B by a specific two-sided enzyme linked immunoassay (Bio-Innovation Ltd, Oxford, UK). The intra- and inter-assay variation for measurements of FSH and Inhibin B were 3% and 4.5%, 15% and 18% respectively. Furthermore, S-albumin (CV 1.8 %), S-Calcium (CV1.2 %), and S-Alkaline Phosphatase (CV 1.3 %) were measured on Cobas c501, and S-Parathyroid hormone (CV 3.4 %) on Cobas e601. All procedures performed as recommended by the manufacturer (Roche). Albumin corrected calcium was calculated (Total S-calcium + (0.0166 x (39,9 g/l - S-albumin (g/l)). All serum assessments were done at the same time to reduce the influence of inter-assay variation.

### Semen analysis

The participants produced a semen sample by masturbation. Self-reported information of duration of ejaculation abstinence was obtained. Trained technicians conducted semen analysis and a detailed description of assessment of semen samples has previously been published. Semen volume was estimated by weighing. For sperm motility assessment, duplicates of 10 µl of well-mixed semen were placed on a glass slide, examined on a heating stage kept at 37 °C, under a microscope at x 400 magnification, and spermatozoa were classified as progressive motile (WHO class A+B), non-progressive motile (class C) or immotile (class D). The average of the two motility assessments was used. For the assessment of the sperm concentration the samples were diluted in a solution of 0.6 mol/l NaHCO3 and 0.4% (v/v) formaldehyde in distilled water, and subsequently assessed using Burker-Turk haemocytometer. Only spermatozoa with tails were counted. Finally, smears were prepared, Papanicolaou stained and spermatozoa morphology assessed according to strict criteria (Menkveld et al., 1990).

### Semen samples for in vitro studies

Semen samples for the in vitro studies were obtained from 40 men from the general population, who were investigated between October 2009 and December 2010. Their semen samples were assessed as described above. To investigate the effect of VD (calcitriol) on sperm motility, the semen samples were exposed to 1nM 1,25(OH)2D3 for 45 minutes and motility scored as described earlier. Motility assessment was conducted in 17 samples (in duplicates) under non-capacitating conditions (in their seminal plasma), and 3 samples were also analyzed in capacitating conditions. 7 consecutively included men were used to investigate a dose-response relationship between VD and sperm motility. Calcium measurements and the acrosome reaction were performed on motile spermatozoa isolated after percoll density gradient centrifugation (Supra sperm gradients, (ORIGO)) following the manufacturer's instructions. Briefly, spermatozoa were separated from seminal plasma by centrifugation on a discontinuous percoll gradient using a two-step gradient comprising 2 ml layers of 55% and 80% percoll respectively. Semen was placed on the top of the gradient and centrifuged at 300 x g for 25 min. The spermatozoa at the base of the 80% fraction were collected and washed with a 5 ml volume of capacitating medium (CaCl2 1,5mM, KCl 4.3 mM, NaHCO3 31mM, HEPES 15mM, Human Serum Albumin 5mg/ml, Glucose 4.5mM, Sodium Pyruvate 0.8mM, Synthetic Serum Replacement, 0.5 ug/ml human recombinant insulin (Sperm preparation media (ORIGO)) and finally resuspended in capacitating media and used for the following assays of calcium measurements and acrosome reaction. All men were part of the above mentioned ongoing surveillance program of semen quality among young Danish men and included after approval from the local ethical committee (No. H-4-2010-016).

### Effect of VD on calcium levels in human spermatozoa

Semen samples were separated by percoll density gradient centrifugation to ensure viable and motile spermatozoa and resuspended in capacitating media for 1.5 hours. Afterwards, loaded with 3 µM of the fluorescent probe (intracellular calcium binding) fura-2/AM (Invitrogen, USA) for 30 min, centrifuged (10 min at 300 g) and washed with Krebs-Ringer buffer (120 mM NaCl, 15 mM NaHCO3, 5 mM KCl, 1 mM CaCl2, 5 mM Na2HPO4 , 1 mM MgCl2 pH 7.3). In a Ca-free Krebs Ringer solution CaCl2 was substituted for 5 mM EGTA (calcium free with EGTA). Subsequently, 80 µl of the sperm sample was diluted in 920 µl Krebs-Ringer buffer and loaded on polylysine-coated chambers to minimize movement of the spermatozoa (Lab-Tek; Nalge Nunc International, USA). A Zeiss Axiovert 135 microscope equipped with a Zeiss Achrostigmat 40×1.3 NA objective was used to acquire images from the fluorescent probe. Excitation was obtained by a Polychrome Villuminator from Till Photonics (Germany) and images were acquired using a Cool Snap CCD camera (Photometrics, USA) from Robert Scientific (Malaysia). For measurements of [Ca2+]i, the excitation wavelengths were 338 and 380 nm, measuring emission above 510 nm using a cut-off filter. Calculations of [Ca2+]i were conducted by MetaFluor software from Molecular Devices and using a Kd of 160nM (Gromada et al., 1996). After background subtraction, ratio images were formed using MetaFluor software. Chemicals, 1α,25(OH)₂D3 (1,25(OH)₂D3), 25-OH D3, progesterone, thapsigargin, nifedipine and ionomycin were purchased from Sigma-Aldrich (USA), PL were carried out in triplicates C-inhibitor U73122 (1-[6-[[17fi-3-methoxyestra-1,3,5(10)-trien-17-yl]amino]hexyl]-1H-pyrrole-2,5-dione) from Biomol, PA, U.S.A. The non-genomic VDR antagonist 1β,25-(OH)₂D3 was a gift from LEO Pharma (Denmark).

All experiments started by using low magnification to ensure visibility of several spermatozoa simultaneously. When a color change was observed in most cells, higher magnification was used. All measurements of [Ca²⁺]ᵢ were based upon evaluation of at least three spermatozoa simultaneously (see exact number in figure legends), and were repeated in at least three separate experiments. We used 10 different semen donors to ensure that all experiments were reproduced on semen samples from at least two different men.

### Acrosome reaction

Acrosome status was assessed according to a modified version of the method described by Cross et al. 1986. Motile spermatozoa were isolated by percoll gradient centrifugation, washed twice with 5 ml 0.9% sodium chloride followed by centrifugation at 300 x g for 10 min. Hereafter, the motile spermatozoa were resuspended with or without 1 nM 1,25(OH)₂D3 for 1.5 hours at room temperature in 0.9% sodium chloride or resuspended in capacitating media for 1.5 hours at 37° C and 5% CO₂ before the sperm was smeared on a glass slide and a cytospin was created. The smear and cytospin were fixed in 96% ethanol for 5 min after air drying, washed in distilled water and stained for minimum 2 hours with Pisum sativum agglutinin labelled with fluorescein isothiocyanate (Sigma Aldrich) in Dulbecco phosphate buffered saline at 4°C. Finally, the slides were washed in distilled water and mounted with Vectrashield (H-1080 Vectar Laboratories) and 200 spermatozoa were counted on each slide using a fluorescence microscope at a magnification of 400x. The mean percentage of acrosome-reacted spermatozoa of the two slides was calculated. When more than half the head of a spermatozoon was brightly and uniformly fluorescent, the acrosome was considered to be intact. Spermatozoa without fluorescence or with a fluorescing band limited to the equatorial segment were considered to be acrosome-reacted. Spermatozoa with a fluorescing band limited to the equatorial segment were named eq-AR. The person assessing the slides was blinded to the treatment of the sample.

### Statistics

The cross sectional cohort of 300 men were stratified according to their 25(OH)D2+3 (25-OH-VD) serum level: < 25nmol/l (deficient), 25-50 nmol/l (insufficient), 50-75 nmol/l (sufficient), and > 75 nmol/l (high). For a basic description median and 5-95 percentiles were used. Between-group differences were tested by the non-parametric Kruskal-Wallis test. Correlations between 25-OH-VD (as a categorical variable) and both semen variables and reproductive hormone levels were examined using the non-parametric Spearman's rank test. To test for the effect of 25-OH-VD, regression analyses were also performed. 25-OH-VD categories were first entered as a co-variate in models having the semen variables or the reproductive hormones as dependent variables. Secondly, 25-OH-VD and 25-OH-VD-squared entered the model as continuous variables. In all regression models, semen volume, sperm concentration, total sperm count, FSH and Inhibin-B were natural logarithm transformed and motility variables logit transformed before analysis to obtain normal distribution of the residuals. The following co-variates were tested: duration of ejaculation abstinence, hour-of-day of blood sampling, smoking, body-mass-index (BMI), age, medication, fever, time from ejaculation to motility assessment, morphology observer, history of cryptorchidism, birth weight, season, albumin-corrected calcium, co-variates with a significance level of 15% or below were included as stated for each analysis in Table 1. The results from the in vitro studies were tested using paired t-test (two-tailed). For all analyses, p<0.05 was considered statistically significant. Analyses were performed using PASW GradPack 18.0 (SPSS Inc., Chicago, IL, USA).

### Example 1

### Vitamin D and intracellular calcium in human spermatozoa

Addition of 1,25(OH)₂D3 (100 pM-10 µM) to the motile spermatozoa induced a rapid increase in intracellular calcium ([Ca2+]i) (Figure 1A). The increase was 5-10 fold from baseline calcium level and started within 5 seconds in the neck region and propagated up in the post-acrosomal region and down through the proximal midpiece (Figure 1A). This effect was reproducible from 100 pM, and the threshold for the observed all-or-nothing response was between 10-50 pM 1,25(OH)₂D3 (Figure 1A). The steep increase in [Ca2+]i was followed by a short plateau phase (up to 50 sec for < 10 nM 1,25(OH)₂D3 ) until [Ca2+]i restored back to baseline concentration (Figure 1A). The amplitude did not increase with higher 1,25(OH)₂D3 concentrations, but the duration of the plateau phase was longer (50 -300 sec). The maximum amplitude (max amplitude) of the calcium increase induced by 1 nM 1,25(OH)₂D3 varied between the investigated men. It was 5-10 fold higher than baseline calcium level and approximately 40-80 % of the max amplitude elicited by 10 µM progesterone. The effect of progesterone could not be abrogated by pre-treatment with 1,25(OH)₂D3 (Figure 1A). The nuclear VDR antagonist (ZK159222, 0.1-100 µM) was unable to abrogate or suppress the effect of 1,25(OH)₂D3 treatment (data not shown), but the VD response was blocked completely by pretreatment with 1-40 µM of the non-genomic VDR antagonist 1β,25(OH)₂D3 (Figure 1B).

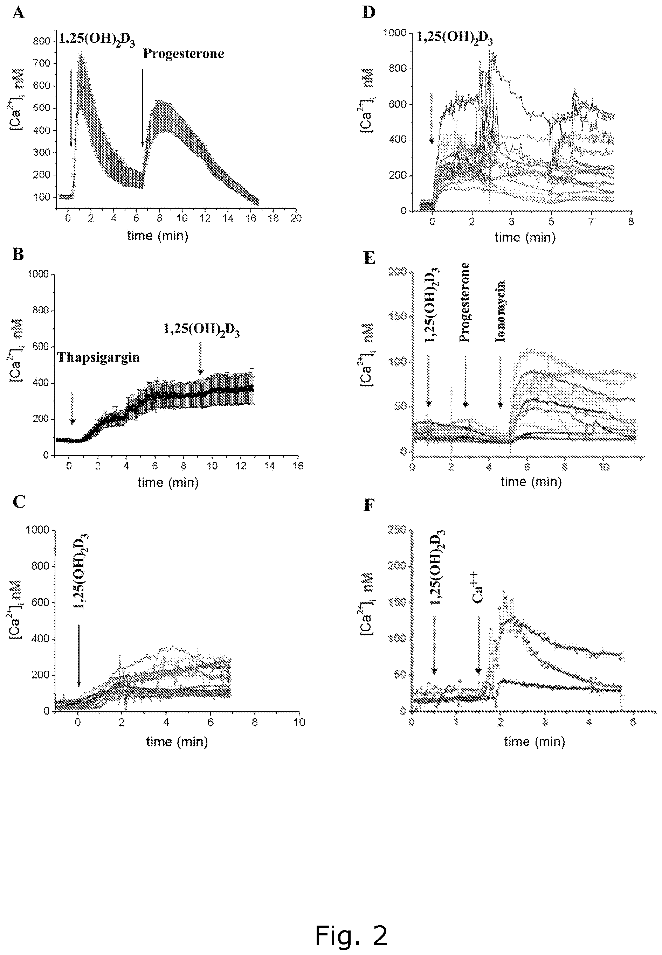

Pretreatment with nifedipine 10 µM did not affect the response to 1,25(OH)₂D3. The calcium increase was initiated rapidly with a maximum amplitude 7 fold higher than baseline, which was followed by 4 minutes long repolarisation phase. However, nifedipine treatment caused an aberrant response to progesterone. The rapid initiation and steep increase in calcium seemed unaffected, but the plateau phase was terminated rapidly by a slow repolarisation phase, and the calcium level was restored to baseline level 9 minutes after initiation of progesterone treatment (Figure 2A). Treatment with thapsigargin 4 µM caused a slow increase in baseline calcium concentration in all spermatozoa (Figure 2B). Nine minutes after thapsigargin exposure it reached a stable plateau 3 fold higher than baseline calcium concentration. Addition of 1,25(OH)₂D3 (9 min after initiation of thapsigargin treatment) to these spermatozoa was unable to elicit a substantial calcium response, while lower concentrations of thapsigargin (< 1 µM) were unable to abrogate the effect of 1,25(OH)₂D3.

To test the mechanism downstream to the VDR, we used a PLC inhibitor U73122, to investigate whether VDR signaling was mediated by a PLC-dependent IP3 production, which may induce an intracellular calcium release. After exposure to 10 µM U73122, the majority (> 80%) of the spermatozoa increased their baseline [Ca²⁺]_{I} more than 5 fold, most were immotile and were unresponsive to treatment with VD. To avoid bias from dying spermatozoa, we changed the concentration of U73122 to 2 µM, where most of the spermatozoa remained low and did not fluctuate in [Ca²⁺]_{I} and were motile. 1,25(OH)₂D3 was unable to induce a rapid increase in [Ca2+]i in spermatozoa exposed to 2 µM U73122. The increase in calcium was instead very slow with long lasting (several minutes) increase unlike the normal rapid response, where the max amplitude was achieved within one minute. The maximum amplitude was also lower, while the plateau phase was longer caused by a delayed repolarisation (Figure 2C). PLC inhibition was unable to abrogate the effect of progesterone (data not shown). We observed a marked response to 1,25(OH)2D3 in a calcium free media (potentially contaminated [Ca²⁺] < 5 uM) without EGTA (Figure 2D). The response to 1,25(OH)₂D3 was aberrant in this media. Here, we observed a longer plateau phase and a delayed repolarisation phase compared to the normal calcium containing media. However, 1,25(OH)₂D3 or progesterone could not induce an increase in [Ca²⁺]ᵢ in calcium free media with 5 µM EGTA, despite the spermatozoa being motile, low in baseline [Ca²⁺]ᵢ and exposed shortly (< 20 min) to low concentrations of EGTA (Figure 2E). Ionomycin (500 nM) induced a low but reproducible increase in [Ca²⁺]ᵢ in the calcium free media with EGTA (Figure 3E). The amplitude of the response to ionomycin was low (< 3 fold from baseline), indicating that the intracellular Ca²⁺ stores could be partially emptied. Addition of 1 mM Calcium to the calcium free media after treatment with either 1,25(OH)₂D3 or progesterone resulted in an increase in [Ca²⁺]_{I}, which was comparable to the increase observed in the normal calcium containing Krebs Ringer solution although the plateau phase was longer in the spermatozoa exposed initially to a calcium free media (Figure 2F). 25-OH D3 (100nM) was unable to elicit an increase in [Ca²⁺]_{I}. Resuspending the motile spermatozoa in non-capacitating media resulted in a lower amplitude to 1 nM 1,25(OH)₂D3 treatment, but a comparable response pattern to the effects observed in capacitating media (data not shown). In both media few spermatozoa were unresponsive to 1,25(OH)₂D₃ treatments.

### Conclusion

These functional studies clearly show that the presence of VDR and the metabolizing enzymes in the neck of human spermatozoa is important for sperm function. Activated VD in physiological concentrations induces a rapid increase in intracellular calcium that last up to 90 seconds before the calcium level is restored back at baseline levels. The effect is mediated through non- genomic VDR activation since 1β, 25 (OH)₂D3 (a competitive antagonist) is able to abrogate the effect of 1,25(OH)₂D3 and the effect is mediated within seconds. The nuclear VDR antagonist zk159222 is unable to abrogate the effect which supports that it is a non-genomic effect of activated vitamin D. The VDR is located in the neck of human spermatozoa where it co-localizes with a calcium storage. The calcium release resulting from treatment with activated vitamin D comes from intracellular storages since the increase also was induced in a media depleted for extracellular calcium and also was present after nifedipine was added to block voltage gated calcium channels. These findings were supported by using thapsigargin that empties intracellular calcium storages and thus were able to diminish the effect of activated vitamin D, which was unable to elicit a calcium increase following thapsigargin treatment.
Together, this shows that activated vitamin D releases calcium from an intracellular calcium storage in the neck of human spermatozoa that propagates of in the head and down the midpiece before calcium levels restore back to baseline within 2 minutes.

This finding is intriguing because a transient calcium increase in the neck of human spermatozoa from intracellular storages has been suggested to be important for capacitation, hyperactivation and the acrosome reaction. There exist multiple compounds and drugs that are able to increase calcium level in human spermatozoa, but there has to our knowledge not been described any drug or compound that can induce a transient calcium increase from the calcium storage in the neck of human spermatozoa. Moreover, the increase in calcium concentration is not dependent on extracellular calcium unlike the progesterone response. Activated vitamin D may thus be a regulator of calcium release from this storage, relevant for sperm function in the female reproductive tract. Progesterone and other compounds can also increase calcium in human spermatozoa, but the increase comes from multiple calcium stores resulting in a higher calcium increase, which is influenced by calcium influx from the extracellular compartment and causes a prolonged calcium increase. The different mechanism of action is important for sperm function, because the high amplitude and prolonged response following progesterone, calcium ionophore and other compounds induces acrosome reaction in most spermatozoa and the spermatozoa become immotile. The transient calcium increase with a modest amplitude elicited by activated vitamin D does not impair sperm motility, which clearly indicates that the mechanism is different and that it has another function for the spermatozoa.

We have investigated expression of VDR and all the VD metabolizing enzymes in the ovary and found that all the proteins are expressed in both granulosa and cumulus cells. Vitamin D is known to be important for folliculogenesis and estrogen production and it has been shown that vitamin D has a cyclic production in the follicular fluid. We believe that activated vitamin D in physological concentrations is likely to have a beneficial effect on the oocyte since it is being produced locally and the progenitors are found in a concentrations comparable to other tissues.

### Example 2

### Vitamin D induces sperm motility and the acrosome reaction in vitro

We investigated a dose-response relationship between 1,25(OH)₂D3 and the induction sperm motility (ABC motility). We tested the effect of 1,25(OH)₂D3 in the concentration range from 10⁻¹⁵M to 10⁻³M and found a non-linear dose-response relationship. We found a modest increase in median sperm motility between 2-7 % in the concentration range from 10⁻¹⁴M to 10⁻⁹M (Figure 3A). The concentration range from 10⁻¹² to 10⁻⁹M induced the highest increase in motility, and the strongest induction was found for 10⁻⁹M with a median increase of 7% (p=0.006). Concentrations above 10⁻⁷M resulted in a decreased motility compared with control (10⁻⁵M, median 15% decrease). We extended this by studying semen samples from 17 men with and without exposure to 10⁻⁹M 1,25(OH)₂D3 for 30 min at room temperature between 1-3 hours after ejaculation. Their mean sperm motility increased significantly (p= 0.010) after VD exposure with 2.9%, 95% CI [1%;5%]. Most men had a modest increase in sperm motility although a subset (n=3) of the men had a slight decrease in motility (Figure 3B). 1,25(OH)₂D3 (10-⁹M) significantly (p=0.024) increased the number of acrosome reacted spermatozoa with an average increase of 6%, 95CI [1%;10%] ( median, control group 12% and VD group 21 %), while the percentage of spermatozoa with an equatorial band (eq.AR) increased from a median of 4.7% to 5.3% (p=0.214) (Figure 3C). 6 men were investigated in non-capacitating conditions (NaCl), and there was no significant difference between VD treated and the control group (4.2% versus 3.7%).

### Conclusion

These *in vitro* data support a positive effect of activated vitamin D on mature spermatozoa. The transient calcium increase induced by activated vitamin D induces sperm motility, which indicates that the spermatozoa improve their chances of reaching the egg. Additionally, activated vitamin D induces the acrosome reaction. The effect on the acrosome reaction is modest, probably because activated vitamin D is unable to release the calcium from the acrosomal calcium storage. Activated vitamin D is only able to induce the acrosome reaction under capacitating conditions, indicating that the calcium increase from the calcium storage in the neck, which propagates up in the head only during activating conditions (capacitation) can release the acrosome and induce the acrosome reaction. This finding is important, because this shows that activated vitamin D during capacitating conditions induces the acrosome reaction, when it is needed for fertilization, but it is unable to induce it during non-capacitating conditions, where the response would be inappropriate.

### Example 3

A permit has been received from the ethical committee to set up a sperm-egg binding test. It is test where obtained eggs that were not fertilized during IVF is used to investigate the ability of spermatozoa to bind to the egg and undergo the acrosome reaction needed for fertilization. These eggs cannot be fertilized, but they are still suitable for testing appropriate sperm-egg interaction. This method will allow to compare both the number of spermatozoa, which are able to bind to the egg, and how many spermatozoa undergoes acrosome reaction after binding, following exposure to media with or without activated vitamin D. The hypothesis is that activated vitamin D in the media will activate the spermatozoa resulting in an increased frequency of spermatozoa binding to the egg and undergoing the acrosome reaction. The outcome of these tests will provide further evidence that the results provided in the examples are indeed indicative of an increased fertilization of an egg, when active vitamin D is supplied according to the invention.

### Example 4

To verify that the transient calcium release in the neck of spermatozoa which can be seen by the addition of active vitamin D is also inducible by analogs of active vitamin D, the following agonist and antagonist have been used that show that VDR-ap generates the response: 1,25(OH)₂D₃, 1β,25(OH)₂D₃, ZK159222, 1,25(OH)₂ Lumisterol₃ (JN) and 25-OHD₃.

### Results

1,25(OH)₂D₃ mediated an instant increase in Ca²⁺ concentration on human spermatozoa (figure 4).

The non-genomic competitive antagonist 1β,25(OH)₂D₃ abrogated the response to 1,25(OH)₂D₃ completely, which indicated that VDR mediated this rapid ionotropic effect through activation of VDR-ap (figure 1D). The fact that 1β,25(OH)₂D₃ did not abrogate the effect of progesterone, indicates the progesterone works through other receptors. (figure 1D).
The genomic VDR antagonist ZK159222 was unable to influence the increase in calcium, which supported a non-genomic effect (figure 5).

The 6-cis-locked agonist 1,25(OH)₂ Lumisterol₃ (JN) is a specific agonist for VDR-ap, to which it binds with the same affinity as 1,25(OH)₂D₃. JN increased [Ca²⁺]ᵢ with similar kinetics as 1,25(OH)₂D₃ in human spermatozoa, which confirmed that VDR-ap mediated the observed ionotropic effect (figure 6).

25-OHD₃ is also a potential agonist for VDR-ap, but 25-OHD₃ (in the nanomolar range) was unable to increase [Ca²⁺]ᵢ substantially, while higher concentrations of 25-OHD₃ induced a marked but delayed increase in [Ca²⁺] (Figure 7). The aberrant response to 25-OHD₃ is not clarified, but could be influenced by the abundant expression of the VD-inactivating enzyme CYP24A1 in the same subcellular compartment or by altered VDR-affinity for 25-OHD₃ in human spermatozoa. The delayed response supports altered kinetics potentially due to metabolic processes since the 1α-hydroxylase is also expressed in the neck region of human sperm. On the other hand, the lower amplitude of the 1,25(OH)₂D₃ mediated response following stimulation with 25-OHD₃ could be due to partial receptor occupancy caused by VDR binding.

These data shows that all agonist which activate the VDR-ap pocket has the capability of inducing the response although compounds that are sensitive to the VD metabolizing enzymes may have a lower potency for eliciting the effects.

### Example 5

We used the Human sperm-zona pellucida interaction test that is designed to determine the capacity of sperm to interact with the zona pellucida (ZP) of human oocytes and serves thus as a test for evaluating fertility potential of human spermatozoa. The tests provide important diagnostic information about sperm fertilising ability and we used a modified version of the test developed by Liu and Baker (Liu and Baker hum reprod 1999 Defective sperm-zona pellucida interaction: a major cause of failure of fertilization in clinical in-vitro fertilization).

### Source of human oocytes:

Oocytes which showed no evidence of two pronuclei or cleavage at 48 to 60 h after insemination in a clinical IVF program were used for the ZP-induced AR tests. If the oocyte had sperm bound to the ZP from the IVF insemination, these were removed by aspiration using a fine glass pipette with an inner diameter (120 µm) slightly smaller than the oocyte diameter. Most of the oocytes were obtained from patients with partial failure of fertilization in standard IVF and more than 50% of these unfertilised oocytes had one or a few sperm penetrating the ZP from the IVF insemination. It is known that oocytes with <10 sperm penetrating the ZP have a similar ability for subsequent sperm-ZP binding and ZP-induced AR as those with no sperm penetration (Liu and Baker 1996). Oocytes with >10 sperm penetrating the ZP were not used. Also degenerate, activated or morphologically abnormal oocytes were not used for the sperm-ZP interaction test. Oocytes were pooled from several patients and used for the test on the same day or kept in the incubator and used within the next 20 days. Between 15-30 oocytes was placed in 1 ml salt solution (storage 2-4 weeks). Salt-stored human oocytes were washed with gamete buffer (COOK) with four changes in 3 hours before they were used for the sperm-egg binding test.

### Sperm analyses and preparation.

Semen samples from five donors were used. One donor with good semen quality (sperm concentration 63 million/ml, 60 % motile sperm), who previously has been responsible for a pregnancy, a young man with good semen quality (sperm concentration 87 million/ml, 58 % motile sperm), two young men with average semen quality (sperm concentration 23/22 million/ml, 42/58 % motile sperm and one man with low semen quality (sperm concentration 1 million/ml, 39 % motile sperm). Each semen sample was divided in two parts. The high quality spermatozoa were selected by percoll gradient centrifugation (55% and 80%). The two different sperm suspensions were washed in supplemented Earles balanced salt solution with 0.3% BSA (sEBSS) and centrifuged at 300 x g for 10 minutes, the supernatant removed and the sperm pellet washed again with sEBSS before centrifugation at 300 x g for 10 minutes. The washed sperm pellet was resuspended with sEBSS to a sperm concentration of 2 x 10⁶/ml for the ZP-interaction test. For the assessment of the sperm concentration the samples were diluted following the manufactures instruction and subsequently determined using SP-100 (Chemometec). For sperm motility assessment, duplicates of 10 µl of well-mixed semen were placed on a glass slide, examined on a heating stage kept at 37 °C, under a microscope at x 400 magnification, and spermatozoa were classified as progressive motile (WHO class A+B), non-progressive motile (class C) or immotile (class D). The average of the two motility assessments was used. To elucidate the impact of activated vitamin D on sperm motility, we added 1 nM activated VD (calcitriol) or vehicle to spermatozoa with or without percoll gradient centrifugation and determined in addition sperm motility after 60 minutes exposure and for some samples after 24 hours at room temperature.

### Sperm-ZP binding

For the sperm-ZP interaction tests, either high quality sperm or total sperm sample (2 x 10⁶) in 0.5 ml of medium (+/- 1 nM Calcitriol) were incubated with three oocytes for 2 h at 37°C in 5% CO₂ in air. After incubation, the oocytes were transferred to sEBSS and washed by repeated aspiration with a glass pipette (inside diameter approximately 250 µm) to dislodge sperm loosely adhering to the surface of the ZP. Sperm binding to all oocytes was assessed. Under these experimental conditions, with the high concentration of sperm in the insemination medium (10 times more than standard IVF insemination), the number of sperm bound tightly to the ZP is normally greater than 100/ZP with sperm from fertile or normospermic men. However, since some donors had low semen quality and thus few high quality sperm isolated after percoll centrifugation, only 0.2-0.5 million/ml was used for the sperm egg binding test in these cases, but the same sperm content was used for all treatments for each patient.

### Results

The number of spermatozoa bound to each oocyte using the test semen from each donor (12 oocytes/batch) varied from 16 to 240. The mean number of spermatozoa bound to each oocyte differed between the different media used and the treatment prior to incubation. Sperm-egg binding was higher in the group of cells exposed to activated vitamin D (mean 74) compared with vehicle treated (mean 52). The sperm bound were significantly higher in the VD treated group compared with the vehicle treated group, mean difference 43% [7%;78%] (p=0.022).The difference in sperm-egg binding between activated vitamin D and vehicle treated cells was found irrespective of isolating high quality spermatozoa by percoll gradient centrifugation prior to incubation of the spermatozoa to the oocyte pool. The difference between VD and vehicle treated sperm was larger in spermatozoa that was not separated by percoll gradient centrifugation (difference 60% in favour of VD treatment, although only borderline significant (p=0.139) due to high variance and low number of investigated subjects). When evaluating VD treatment in sperm isolated with percoll gradient centrifugation the same trend was found since VD treated cells had an improved sperm-egg binding in average 28 % [5%;50%] (p=0.027) higher than vehicle treated spermatozoa (Figure 8) The same trend towards improved sperm egg binding after activated VD treatment was present irrespective of pre-treatment of the spermatozoa. It was not only mean sperm egg binding that was higher after VD treatment, Sperm egg binding was also higher following VD treatment in each of the 5 semen donors as shown in Table 2.

**Table 2: Mean sperm-egg binding in 5 patients stratified according to pretreatment and exposure to activated vitamin D ((1,25(OH)2D3)).**

| | Sperm bound (N) | Sperm bound (N) | Sperm bound (N) | Sperm bound (N) |
|---|---|---|---|---|
| Percoll centrifugation | NO | NO | YES | YES |
| 1,25(OH)2D3 | NO | YES | NO | YES |
| Patient 1 | 108 | 195 | 52 | 71 |
| Patient 2 | 55 | 64 | 86 | 116 |
| Patient 3 | 20 | 16 | 36 | 48 |
| Patient 4 | 30 | 50 | 60 | 70 |
| Patient 5 | 28 | 61 | 35 | 38 |

The patient with low semen quality (patient 3 in Table 2) had a marked increase in sperm-egg binding after VD treatment following percoll gradient centrifugation (increase 33%), while there was a modest decline in sperm-egg binding in spermatozoa that was not separated by Percoll centrifugation. The remaining four patients had effects of VD irrespective of which pre-treatment (with and without Percoll gradient centrifugation) of the sperm (Table 2).

Motility assessment following exposure to 1 nM of activated vitamin D in spermatozoa isolated by percoll gradient centrifugation showed an increase in sperm motility from 62 % to 65 % motile sperm after 60 minutes exposure to activated vitamin D. Sperm motility was not higher after exposure to activated VD (mean 48) when spermatozoa with no percoll centrifugation were investigated than with vehicle treatment (mean 50). The same pattern was found for the proportion of progressive motile spermatozoa. The observed differences in sperm motility are therefore unlike to explain the marked effect of activated vitamin D on sperm-egg binding. It is known that agents that mediate calcium increase in human spermatozoa such as calcium ionophore A23187 inhibits sperm-egg interaction and thus sperm egg binding due to premature acrosome reaction and secondary immotility of the spermatozoa (Liu and Baker J. Reprod Fert 1990). We tested whether VD treatment caused immotility in the treated spermatozoa by reevaluating spermatozoa after 24 hours exposure at room temperature to 1 nM of calcitriol. We found a modest decrease in the proportion of motile sperm both with and without percoll gradient centrifugation prior to analyses, but the decrease in sperm motility did not differ substantially from vehicle treated spermatozoa (Figure 9).

### Conclusion

The observed differences show that spermatozoa have an improved sperm-egg binding following treatment with activated VD during incubation of the sperm and the oocytes. The improved sperm-egg binding following VD treatment was in average 43% and was present irrespective of percoll gradient centrifugation prior to incubation, although the effect was most reliable in high quality spermatozoa separated by percoll gradient centrifugation. All the patients irrespective of semen quality had a positive response to activated VD. A patient with severe oligospermia had only few motile spermatozoa befoe inoculation with the eggs, which may explain the blunted response when using non-isolated sperm. Since the same donor had a marked response after isolation of the motile sperm by percoll gradient centrifugation. All the patients had a positive effect of activated VD after percoll gradient centrifugation, which imply that the response is higher when the spermatozoa are viable, motile and have normal morphology. Activated vitamin D induced a modest increase in sperm motility especially in sperm isolated after percoll gradient centrifugation, but the improved sperm-egg binding cannot solely be explained by improved sperm motility. Underpinned by the highest increase in sperm-egg binding in sperm with no percoll centrifugation, where there was no net increase in sperm motility. The observed findings indicate that all agonists for VDR-ap have the potential to improve sperm-egg binding in a comparable way, although there may be differences in potency between the agonists. The improved binding to the oocyte may be mediated by an intracellular calcium increase, but the VD mediated calcium increase differs from other compounds known to increase calcium concentration such as calcium ionophores that result in lower egg binding and immotility of the spermatozoa. The calcium release mediated by activated VD differs since the spermatozoa remain motile even after 24 hours exposure and sperm egg binding improves significantly following exposure during inoculation.

### Example 6

Human sperm-zona pellucida interaction test was used to investigate the effect of Vitamin D analogs since the test is designed to determine the capacity of sperm to interact with the zona pellucida (ZP) of human oocytes and serves thus as a test for evaluating fertility potential of human spermatozoa.

### Source of human oocytes:

Oocytes which showed no evidence of two pronuclei or cleavage at 48 to 60 h after insemination in a clinical IVF program were used for the ZP-induced AR tests. If the oocyte had sperm bound to the ZP from the IVF insemination, these were removed by aspiration using a fine glass pipette with an inner diameter (120 µm) slightly smaller than the oocyte diameter. Most of the oocytes were obtained from patients with partial failure of fertilization in standard IVF and more than 50% of these unfertilised oocytes had one or a few sperm penetrating the ZP from the IVF insemination. It is known that oocytes with <10 sperm penetrating the ZP have a similar ability for subsequent sperm-ZP binding and ZP-induced AR as those with no sperm penetration (Liu and Baker 1996). Oocytes with >10 sperm penetrating the ZP were not used. Also degenerate, activated or morphologically abnormal oocytes were not used for the sperm-ZP interaction test. Oocytes were pooled from several patients and used for the test on the same day or kept in the incubator and used within the next 20 days. Between 15-30 oocytes was placed in 1 ml salt solution (storage 2-4 weeks). Salt-stored human oocytes were washed with gamete buffer (COOK) with four changes in 3 hours before they were used for the sperm-egg binding test.

### Sperm analyses and preparation.

Semen sample from 1 donor was used. One donor with good semen quality (sperm concentration 205 million/ml, 34 % motile sperm). The semen sample was divided in two parts. The high quality spermatozoa were selected by percoll gradient centrifugation (55% and 80%). The two different sperm suspensions were washed in supplemented Earles balanced salt solution with 0.3% BSA (sEBSS) and centrifuged at 300 x g for 10 minutes, the supernatant removed and the sperm pellet washed again with sEBSS before centrifugation at 300 x g for 10 minutes. The washed sperm pellet was resuspended with sEBSS to a sperm concentration of 2 x 106/ml for the ZP-interaction test. For the assessment of the sperm concentration the samples were diluted following the manufactures instruction and subsequently determined using SP-100 (Chemometec). For sperm motility assessment, duplicates of 10 µl of well-mixed semen were placed on a glass slide, examined on a heating stage kept at 37 °C, under a microscope at x 400 magnification, and spermatozoa were classified as progressive motile (WHO class A+B), non-progressive motile (class C) or immotile (class D). The average of the two motility assessments was used. To elucidate the impact of the active vitamin D analog 1α,25(OH)2-lumisterol (JN) on sperm motility, we added 10 nM JN or vehicle to spermatozoa with or without percoll gradient centrifugation and determined in addition sperm motility after 60 minutes exposure and for some samples after 24 hours at room temperature.

### Sperm-ZP binding

For the sperm-ZP interaction tests, either high quality sperm or total sperm sample (2 x 106) in 0.5 ml of medium (+/- 10 nM 1α,25(OH)2-lumisterol (JN)) were incubated with four oocytes for 2 h at 37oC in 5% CO2 in air. After incubation, the oocytes were transferred to sEBSS and washed by repeated aspiration with a glass pipette (inside diameter approximately 250 µm) to dislodge sperm loosely adhering to the surface of the ZP. Sperm binding to all oocytes was assessed. Under these experimental conditions, with the high concentration of sperm in the insemination medium (10 times more than standard IVF insemination), the number of sperm bound tightly to the ZP is normally achieved easily with sperm from fertile or normospermic men.

### Results

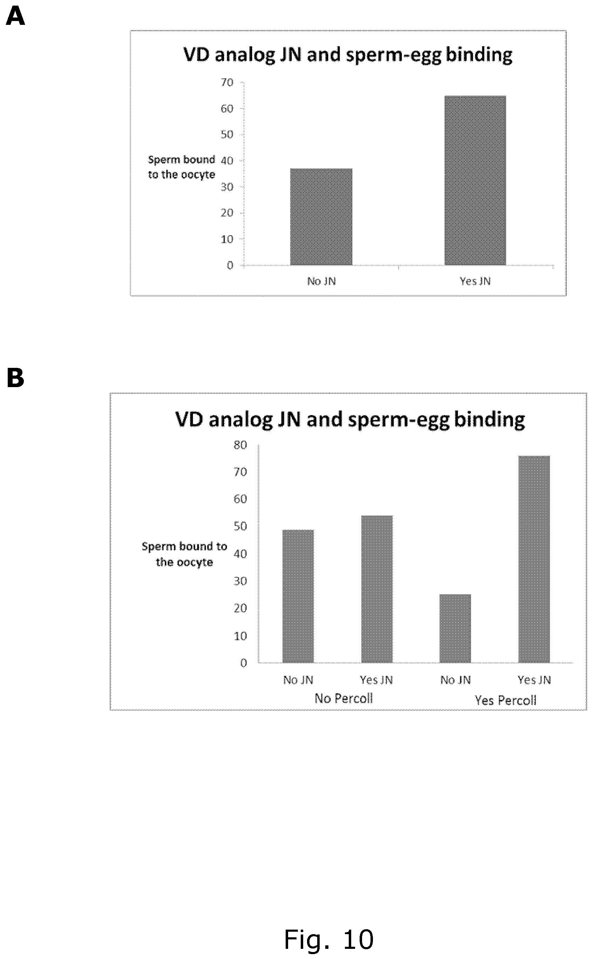

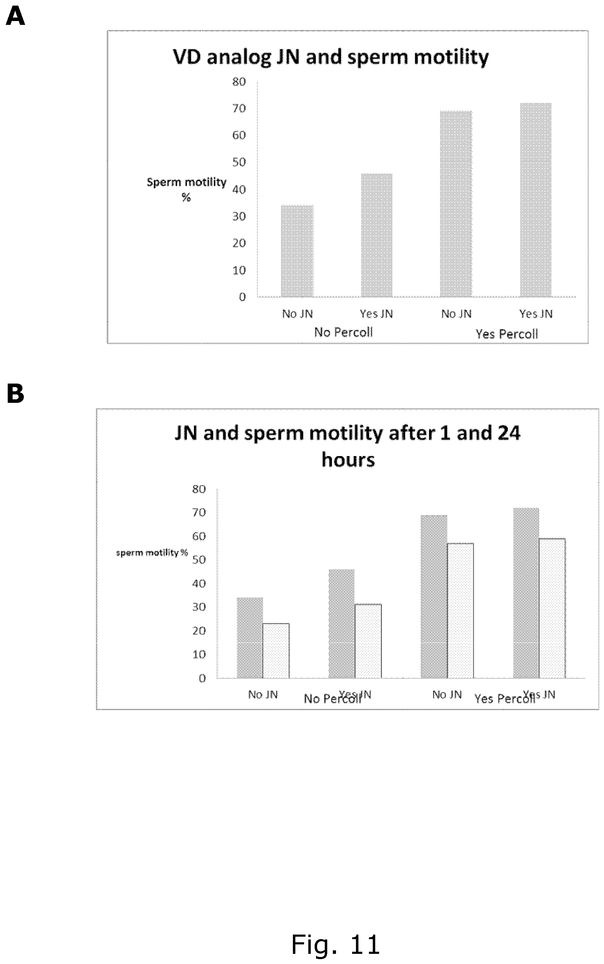

The number of spermatozoa bound to each oocyte using the test varied from 7 to 103. The mean number of spermatozoa bound to each oocyte differed between the different media used and the treatment prior to incubation. Sperm-egg binding was higher in the group of cells exposed to 1α,25(OH)2-lumisterol (JN) (mean 65) compared with vehicle treated (mean 37) (Figure 10A). The difference in sperm-egg binding between JN and vehicle treated cells was found irrespective of isolating high quality spermatozoa by percoll gradient centrifugation prior to incubation of the spermatozoa to the oocyte pool. The difference between VD and vehicle treated sperm was larger in spermatozoa that was separated by percoll gradient centrifugation (difference 300% in favour of JN treatment). When evaluating JN treatment in sperm isolated without percoll gradient centrifugation the same trend was found since VD treated cells had an improved sperm-egg binding in average 11 % higher than vehicle treated spermatozoa (Figure 10B) The same trend towards improved sperm egg binding after activated VD treatment was present irrespective of pre-treatment of the spermatozoa. Motility assessment following exposure to 10 nM of JN in spermatozoa isolated by percoll gradient centrifugation showed an increase in sperm motility from 69 % to 72 % motile sperm after 60 minutes exposure to JN. Sperm motility was also higher after exposure to JN (mean 46) when spermatozoa with no percoll centrifugation were investigated than with vehicle treatment (mean 34) (Figure Figure 11A). The same pattern was found for the proportion of progressive motile spermatozoa. The observed differences in sperm motility are therefore unlike to explain the marked effect of activated vitamin D on sperm-egg binding. It is known that agents that mediate calcium increase in human spermatozoa such as calcium ionophore A23187 inhibits sperm-egg interaction and thus sperm egg binding due to premature acrosome reaction and secondary immotility of the spermatozoa (Liu and Baker J. Reprod Fert 1990). We tested whether VD treatment caused immotility in the treated spermatozoa by reevaluating spermatozoa after 24 hours exposure at room temperature to 10 nM of JN. We found a modest decrease in the proportion of motile sperm both with and without percoll gradient centrifugation prior to analyses, but the decrease in sperm motility did not differ substantially from vehicle treated spermatozoa (Figure 11B).

### Conclusion

The observed differences show that spermatozoa have an improved sperm-egg binding following treatment with JN during incubation of the sperm and the oocytes. This is in line with the improved sperm-egg binding following VD treatment, which also was present irrespective of percoll gradient centrifugation prior to incubation. Although the effect with activated VD was also most reliable in high quality spermatozoa separated by percoll gradient centrifugation as for JN. Jn induced a modest increase in sperm motility especially in sperm with no percoll gradient centrifugation, but the improved sperm-egg binding cannot solely be explained by improved sperm motility. Underpinned by the highest increase in sperm-egg binding in sperm with percoll centrifugation, where there was a limited increase in sperm motility. The observed findings indicate that all agonists for VDR-ap have the potential to improve sperm-egg binding in a comparable way, although there may be differences in potency between the agonists. The improved binding to the oocyte may be mediated by an intracellular calcium increase, but the VD mediated calcium increase differs from other compounds known to increase calcium concentration such as calcium ionophores that result in lower egg binding and immotility of the spermatozoa. The calcium release mediated by JN differs since the spermatozoa remain motile even after 24 hours exposure and sperm egg binding improves significantly following exposure during inoculation.

## Claims

1. Use of a composition comprising calcitriol or analogs thereof for, in vitro, improving the ability of a spermatozoa to bind to an egg;
wherein said analogs are agonists which can bind and release the non-genomic effect via VDR (Vitamin D receptor) in spermatozoa OR wherein said analogs are selected from the group consisting of the list of table 1 and 1,25(OH)₂ Lumisterol₃ (JN) and 25-OHD₃;
wherein calcitriol or analogs thereof is present at a concentration of 10⁻¹⁵ 10⁻⁴ M.

2. The use according to claim 1, wherein said analogs are selected from the group consisting of the list of table 1 and 1,25(OH)₂ Lumisterol₃ (JN) and 25-OHD₃;

3. The use according to claim 1, wherein said composition comprises calcitriol.

4. The use according to any of claims 1-3, wherein the increase in sperm-egg binding is in the range 10-200% compared to untreated samples.

5. The use according to any of claims 1-3, wherein the spermatozoa are provided as a purified sample of spermatozoa.

6. The use according to claim 5, wherein the spermatozoa sample is purified by a method selected from the group consisting of density-gradient-centrifugation, spin-up, glass-wool columns, simple centrifugation, percoll gradient, and simple washing.

7. The use according to any of claims 1-6, wherein the spermatozoa are of mammalian origin, such as human, horse, pig, cow or sheep.

8. The use according to any of claims 1-7, wherein calcitriol or analogs thereof is present at a concentration of 10⁻¹⁴- 10⁻⁸ M.

9. A method for improving the ability of a spermatozoa to fertilize an egg in vitro, said method comprising
- providing a medium comprising calcitriol or analogs thereof,
- providing a sample of spermatozoa, and
- incubating said sample of spermatozoa with said medium.
wherein said improved ability of a spermatozoa to fertilize an egg is at least partly due to an increased binding of spermatozoa to the egg;
wherein said analogs are agonists which can bind and release the non-genomic effect via VDR (Vitamin D receptor) in spermatozoa OR wherein said analogs are selected from the group consisting of the list of table 1 and 1,25(OH)₂ Lumisterol₃ (JN) and 25-OHD₃;
wherein calcitriol or analogs thereof is present at a concentration of 10⁻¹⁵ - 10⁻⁴ M.

10. The method according to claim 9, wherein said analogs are selected from the group consisting of the list of table 1 and 1,25(OH)₂ Lumisterol₃ (JN) and 25-OHD₃;

11. The method according to claim 9, wherein said composition comprises calcitriol.

## Patentansprüche

1. Verwendung einer Zusammensetzung umfassend Calcitriol oder Analoga davon, in vitro, zur Verbesserung der Fähigkeit von Spermien, sich an eine Eizelle zu binden;
wobei die Analoga Agonisten sind, die sich binden und über VDR (Vitamin-D-Rezeptor) in Spermien die nicht-genomische Wirkung freisetzen können ODER wobei die Analoga ausgewählt sind aus der Gruppe bestehend aus der Liste von Tabelle 1 und 1,25(OH)₂-Lumisterol₃ (JN) und 25-OHD₃;
wobei Calcitriol oder Analoga davon in einer Konzentration von 10⁻¹⁵-10⁻⁴ M vorhanden sind.

2. Verwendung nach Anspruch 1, wobei die Analoga ausgewählt sind aus der Gruppe bestehend aus der Liste von Tabelle 1 und 1,25(OH)₂-Lumisterol₃ (JN) und 25-OHD₃.

3. Verwendung nach Anspruch 1, wobei die Zusammensetzung Calcitriol umfasst.

4. Verwendung nach einem der Ansprüche 1-3, wobei die Zunahme der Sperma-Eizellen-Bindung im Bereich von 10-200 % im Vergleich zu unbehandelten Proben liegt.

5. Verwendung nach einem der Ansprüche 1-3, wobei die Spermien als eine gereinigte Probe von Spermien bereitgestellt sind.

6. Verwendung nach Anspruch 5, wobei die Spermienprobe durch ein Verfahren gereinigt ist, das aus der Gruppe bestehend aus Dichtegradienenzentrifugation, Hochzentrifugation (Spin-up), Glaswollesäulen, einfacher Zentrifugation, Percoll-Gradienten und einfachem Waschen ausgewählt ist.

7. Verwendung nach einem der Ansprüche 1-6, wobei die Spermien von Säugern stammen, wie etwa Mensch, Pferd, Schwein, Rind oder Schaf.

8. Verwendung nach einem der Ansprüche 1-7, wobei Calcitriol oder Analoga davon in einer Konzentration von 10⁻¹⁴-10⁻⁸ M vorhanden sind.

9. Verfahren zum Verbessern der Fähigkeit von Spermien, eine Eizelle in vitro zu befruchten, wobei das Verfahren Folgendes umfasst:
- Bereitstellen eines Mediums, das Calcitriol oder Analoga davon umfasst,
- Bereitstellen einer Probe von Spermien, und
- Inkubieren der Probe von Spermien mit dem Medium,
wobei die verbesserte Fähigkeit von Spermien, eine Eizelle zu befruchten, zumindest teilweise auf eine erhöhte Bindung von Spermien mit der Eizelle zurückzuführen ist;
wobei die Analoga Agonisten sind, die sich binden und über VDR (Vitamin-D-Rezeptor) in Spermien die nicht-genomische Wirkung freisetzen können ODER wobei die Analoga ausgewählt sind aus der Gruppe bestehend aus der Liste von Tabelle 1 und 1,25(OH)₂-Lumisterol₃ (JN) und 25-OHD₃;
wobei Calcitriol oder Analoga davon in einer Konzentration von 10⁻¹⁵-10⁻⁴ M vorhanden sind.

10. Verfahren nach Anspruch 9, wobei die Analoga ausgewählt sind aus der Gruppe bestehend aus der Liste von Tabelle 1 und 1,25(OH)₂-Lumisterol₃ (JN) und 25-OHD₃.

11. Verfahren nach Anspruch 9, wobei die Zusammensetzung Calcitriol umfasst.

## Revendications

1. Utilisation d'une composition comprenant le calcitriol ou des analogues de celui-ci, in vitro, pour améliorer l'aptitude d'un spermatozoïde à se lier à un ovule ;
lesdits analogues étant des agonistes qui peuvent se lier et libérer l'effet non génomique par l'intermédiaire du VDR (récepteur de la vitamine D) dans les spermatozoïdes OU lesdits analogues étant choisis dans le groupe constitué par la liste du tableau 1 et 1,25(OH)₂ lumistérol₃ (JN) et 25-OHD₃ ;
ledit calcitriol ou lesdits analogues de celui-ci étant présents à une concentration de 10⁻¹⁵ à 10⁻⁴ M.

2. Utilisation selon la revendication 1, lesdits analogues étant choisis dans le groupe constitué par la liste du tableau 1 et 1,25(OH)₂ lumistérol₃ (JN) et 25-OHD₃.

3. Utilisation selon la revendication 1, ladite composition comprenant le calcitriol.

4. Utilisation selon l'une quelconque des revendications 1 à 3, l'augmentation dans la liaison sperme-ovule se trouvant dans la plage de 10 à 200 % par rapport à des échantillons non traités.

5. Utilisation selon l'une quelconque des revendications 1 à 3, lesdits spermatozoïdes étant fournis sous la forme d'un échantillon purifié de spermatozoïdes.

6. Utilisation selon la revendication 5, ledit échantillon de spermatozoïdes étant purifié par un procédé choisi dans le groupe constitué par une centrifugation à gradient de densité, une mise en rotation (spin-up), des colonnes à laine de verre, une centrifugation simple, un gradient de Percoll et un lavage simple.

7. Utilisation selon l'une quelconque des revendications 1 à 6, lesdits spermatozoïdes provenant d'un mammifère, tel que l'homme, un cheval, un porc, une vache ou un mouton.

8. Utilisation selon l'une quelconque des revendications 1 à 7, ledit calcitriol ou lesdits analogues de celui-ci étant présents à une concentration de 10⁻¹⁴ à 10⁻⁸ M.

9. Procédé pour améliorer l'aptitude d'un spermatozoïde à fertiliser un ovule in vitro, ledit procédé comprenant
- la fourniture d'un milieu comprenant le calcitriol ou des analogues de ceux-ci,
- la fourniture d'un échantillon de spermatozoïdes, et
- l'incubation dudit échantillon de spermatozoïdes avec ledit milieu ;
ladite aptitude améliorée d'un spermatozoïde à fertiliser un ovule étant au moins en partie due à une liaison accrue des spermatozoïdes à l'ovule ;
lesdits analogues étant des agonistes qui peuvent se lier et libérer l'effet non génomique par l'intermédiaire du VDR (récepteur de la vitamine D) dans les spermatozoïdes OU lesdits analogues étant choisis dans le groupe constitué par la liste du tableau 1 et 1,25(OH)₂ lumistérol₃ (JN) et 25-OHD₃ ;
ledit calcitriol ou lesdits analogues de celui-ci étant présents à une concentration de 10⁻¹⁵ à 10⁻⁴ M.

10. Procédé selon la revendication 9, lesdits analogues étant choisis dans le groupe constitué par la liste du tableau 1 et 1,25(OH)₂ lumistérol₃ (JN) et 25-OHD₃.

11. Procédé selon la revendication 9, ladite composition comprenant le calcitriol.
